# EUROPEAN PATENT APPLICATION

(11) **EP 1 113 082 A2**
(43) Date of publication of application: **04.07.2001**
(21) Application number: 00126204.7
(22) Date of filing: 19.12.1990
(51) Int. Cl.: C12Q 1/70

(54) **Amplification capture assay**

(30) Priority: 29.12.1989 US 459030
(62) Divisional of application: 90124738.7
(71) Applicant: ENZO BIOCHEM, INC., New York, N.Y. 10013 (US)
(72) Inventor: Brakel, Christine L., Brightwaters, NY 11718 (US); Spadoro, Joanne P., Nutley, NJ 07110 (US)
(74) Representative: VOSSIUS & PARTNER

(57) **Abstract**

Disclosed are compositions for the determination of complementary single-stranded target nucleic acids in a sample to be tested. Also disclosed are methods for the detection of single-stranded target genetic material in a sample to be tested which use the compositions of the invention.

## Description

The present invention relates to the field of hybridization assays for the determination of specific nucleic acids in a sample. More particularly, it relates to improvements in combining amplification procedures and multiprobe hybridization assays.

The development of specific binding assay techniques has provided extremely useful analytical methods for determining various substances of diagnostic, medical, environmental and industrial importance which appear in various liquid or solid (e.g., tissue) samples at very low concentrations. Specific binding assays are based on the specific interaction between a bindable analyte under determination and a binding partner therefor, i.e. analyte-specific moiety. The binding of the analyte-specific moiety and interaction of any additional reagents, if necessary, effect a mechanical separation of bound and unbound labeled analyte or affect the label in such a way as to modulate the detectable signal. The former situation is normally referred to as heterogeneous and the latter as homogeneous, in that the latter does not require a separation step.

Where either the analyte or its binding partner is a target nucleic acid sequence and the other is a complementary nucleic acid sequence, the assay is known as a nucleic acid hybridization assay. See, generally, Falkow et al, U.S. Patent No. 4,358,535; Kourilsky et al, U.S. Patent No. 4,581,333; Albarella et al, U.S. Patent No.4,563,417 and Paau et al, U.S. Patent No. 4,556,643.

In conventional label conjugate specific binding assay techniques, a sample of the liquid medium to be assayed is combined with various reagent compositions. Such compositions include a label conjugate comprising a binding component incorporated with a label. The binding component in the conjugate participates with other constituents, if any, of the reagent composition and the ligand in the medium under assay to form a binding reaction system producing two species or forms of the conjugate, e.g., a bound-species (conjugate complex) and a free-species. In the bound-species, the binding component of the conjugate is bound by a corresponding binding partner whereas in the free species, the binding component is not so bound. The amount or proportion of the conjugate that results in the bound species compared to the free species is a function of the presence (or amount) of the analyte to be detected in the test sample.

An alternative format for specific binding assays is the "sandwich" assay protocol in which the target analyte is bound between a first specific binding partner, which is fixed directly or through a linkage group to a solid matrix, and a second specific binding partner, which is associated with a signal generating or labeling system. When both the first and second specific binding partners are oligo- or polynucleotides and the target is a nucleic acid, the assay is known as a sandwich hybridization assay See, generally, Ranki et al, U.S. Patent No. 4,486,539 and Ranki et al, U.S. Patent No.4,563,419. See also, Virtanen et al., Novel test for Viral diagnosis; Detection of adenovirus in nasopharyngeal mucus aspirates by means of nucleic-acid sandwich hybridisation, Lancet, 1:381-383 (1983) and Ranki et al., Sandwich hybridization as a convenient method for the detection of nucleic acid in crude samples, Gene, 21:77-85 (1983).

Urdea et al., A novel method for the rapid detection of specific nucleotide sequences in crude biological samples without blotting or radioactivity; application to the analysis of hepatitis B virus in human serum, Gene, 61:253-264 (1987) describes an in solution hybridization which utilizes a multiplicity of soluble oligonucleotide probes. The method disclosed therein uses an in solution hybridization with two sets of probes comprised of 48 different hepatitis virus-specific sequences. One set of probes (the A probe set) has a nucleotide sequence comprised of a target-specific region and a non-homopolymeric capture-specific region. The non-homopolymeric capture-specific region of this probe set is complementary to a capturing nucleotide sequence that is labeled with biotin and is bindable by avidin-modified beads. The other set of probes (the B probe set) has a nucleotide sequence comprised of a target-specific region and another non-homopolymeric non-target-specific region. This non-homopolymeric non-target-specific region is complementary to a label capturing nucleotide multimer sequence. The label capturing nucleotide multimer sequence itself has a region of complementarity to the non-homopolymeric non-target-specific region as well as a region complementary to a signaling oligonucleotide that is labeled with the signaling agent, horseradish peroxidase. Following in solution hybridization of target nucleic acids with probe sets A and B, the hybridized complex is then hybridized to the biotin-labeled capture probe, which in turn is then bound by the avidin-modified beads. The matrix bound complex is allowed to hybridize with the label capturing nucleotide multimer sequence and then with the signaling oligonucleotide. The amount of bound hybridized complex is determined by the presence of horseradish peroxidase. The method disclosed in this work required synthesis of 51 different oligonucleotides: 48 different target-specific probes with their attached non-homopolymeric regions, the capturing probe, the label capturing nucleotide multimer, and the signaling probe. It also required the modification of the latter three probes with biotin, with crosslinking reagents and with horseradish peroxidase, respectively. The authors demonstrate a sensitivity that was proportional to the number of different probes.

Zivin and Monahan, EPO Publ. No. 0 305 145, published March 1, 1989, discloses an in solution sandwich hybridization method which utilizes two soluble oligonucleotide probes. One of the probes has a homopolynucleotide tail which is captured by a solid support coated with a complementary homopolynucleotide and the other probe is a detectable probe complementary to a different region of the target. A target nucleic acid sequence is permitted to hybridize in solution with the two probes. Only after hybridization is complete is the sample containing the hybridized complex brought into contact with and captured by an insoluble solid support. Commonly assigned, co-pending U.S. patent application serial no. 005,377, filed January 15, 1987 also discloses sandwich hybridization and is incorporated herein by reference.

Several early methodologies for the amplification of nucleic acids are known. See, for example, Olson, et al., Enzymatic Amplification of a Chemically Synthesized DNA Fragment, Nucleic Acids Research, 243-60 (1975);Panet and Khorana, Studies on Polynucleotides. The Linkage of Deoxyribopolynucleotide to Cellulose and Its Use in their Replication, J. Biol. Chem., 249:5213-5221 (1974); Gefter, et al., DNA Synthesis in Cell Free Extracts, Part 3: Catalytic Properties of DNA Polymerase II, J. Biol. Chem., 247:3321-3326 (1972): and Kleppe, et al., Studies on Polynucleotides, part 96. Repair replication of short synthetic DNAs as catalyzed by DNA polymerases, J. Mol. Biol., 56:341-361 (1971).

Another, more recent, methodology is the polymerase chain reaction (PCR) procedure, which has become a valuable technique to make available larger quantities of nucleic acids naturally occuring in minute amounts. Briefly, the PCR procedure is as follows. Separate (single - stranded) complementary strands of a target to be copied are each treated with a molar excess of oligonucleotide primers complementary to opposite ends of the two strands (e.g., each primer is a 5'-primer). If the original target is double-stranded it must first be rendered single-stranded. The primers are extended by adding individual nucleotides and an appropriate polymerase or reverse transcriptase to form complementary primer extension products. Once the primer extension product is formed, each of the duplicate double-stranded intermediate products is melted (denatured) to single-strand form. The extension product synthesized from one primer when separated from its complement and hybridized to the other primer becomes a template for synthesis of the extension product of the other primer. See, Mullis et al., U.S. Patent Nos. 4,683,195 and 4,683,202.

In most applications of PCR, the amplified DNAs are analyzed by Southern or dot blot hybridization to radiolabeled or biotin labeled probes. See, for example, Ou et al., DNA amplification for direct detection of HIV-1 in DNA of peripheral blood mononuclear cells, Science, 239:292-295 (1985); Saiki et al., Primer-directed enzymatic amplification of DNA with a thermostable DNA polymerase, Science, 239:487-491 (1988); Higuchi et al., DNA typing from single hairs, Nature, 332:543-546 (1988); and Bugawan et al., The use of non-radioactive oligonucleotide probes to analyze enzymatically amplified DNA for prenatal diagnosis and forensic HLA typing,

Biotechnology, 6:943-947 (1988). Because such procedures can take 2-3 days to complete, they are generally unsuitable for use in high volume applications, clinical-testing situations and, are particularly unsuitable for application to automated instrumentation.

Describing another amplification system, Lizardi et al., Exponential Amplification of Recombinant-RNA Hybridization Probes, Biotechnology, 6:1197-1202 (1988) report having synthesized recombinant RNA molecules that function both as hybridization probes and as templates for exponential amplification by QBeta replicase. Each recombinant consists of a sequence specific for the protozoan parasite, Plasmodium falciparum, embedded within the sequence of MDV-1 RNA, which is a natural template for QBeta replicase. The probe sequence was inserted within a hairpin loop that occurs on the exterior of MDV-1 RNA. The recombinant RNAs hybridize specifically to complementary DNA, despite topological constraints on the probe domain, are replicated at the same rate as the MDV-1 RNA, despite their additional length, and are able to serve as templates for the synthesis of a large number of RNA copies. The results are said to demonstrate the feasibility of employing exponentially replicable RNAs in bioassays where they would serve the dual role of specific probe and amplifiable reporter. These assays may suffer from non-target-specific amplification of non-specifically hybridized or trapped dual role probe/amplifiable reporter.

Kemp et al, Colorimetric detection of specific DNA segments amplified by polymerase chain reactions, Proc. Natl. Acad. Sci. USA, 86:2423-2427 (1989) describes an assay for colorimetric detection of amplified DNA. The target DNA is first amplified by PCR, and then a second set of oligonucleotides, nested between the first two, is incorporated by three or more PCR cycles. These oligonucleotides bear ligands: for example, one can be biotinylated and the other can contain a site for a double-stranded DNA-binding protein. After linkage to an immobilized affinity reagent (such as a cloned DNA binding protein) and labeling with a second affinity reagent (for example, avidin) linked to horseradish peroxidase, reaction with a chromogenic substrate allows detection of the amplified DNA.

Keller and Khan, Identification of HIV sequences using nucleic acid probes, Am. Clinical Labs, (November 1988) discusses recently developed methods for the detection of HIV-1 viral nucleic acid. One method described begins by using polymerase chain reaction on HIV-1 provirus which exists either as integrated or episomal DNA. They note that viral RNA can also be specifically amplified with some additional steps, citing Saiki et al., supra. The RNA template is either viral mRNA or packaged virion RNA. A cDNA copy of the viral RNA is synthesized using the PCR primers and reverse transcriptase. The authors indicate that the detection formats should probably be modified to provide simpler but more reliable tests. Sandwich hybridization is mentioned as an alternative and a conventional sandwich hybridization scheme is illustrated but no specifications are provided.

Kwoh et al., Transcription-based amplification system and detection of amplified human immunodeficiency virus type 1 with a bead-based sandwich hybridization format, Proc. Natl. Acad. Sci. USA, 86:1173-1177 (1989) discloses a transcription-based amplification system (TAS). Each cycle is composed of two steps. The first is a cDNA synthesis step that produces one copy of a double-stranded DNA template for each copy of RNA or DNA target nucleic acid. During the course of this cDNA synthesis step, a sequence recognized by a DNA-dependent RNA polymerase is inserted into the cDNA copy of the target to be amplified. The second step is the amplification of the target sequence by the transcription of the cDNA template into multiple copies of RNA. This procedure has been applied to the detection of HIV-1. Detection of the TAS-generated RNA from HIV-1-infected cells can be accomplished by a bead-based sandwich hybridization protocol. Sephacryl beads containing oligonucleotide were prepared as described in Ghosh and Musso, Covalent attachment of oligonucleotides to solid supports, Nucleic Acids Research,15:5353-5372 (1987). This bead-bound oligonucleotide was used to capture the TAS-amplified HIV-1 sequences after these targets had hybridized to a ³²P-labeled detection oligonucleotide.

Notwithstanding these efforts, much room for improvement remains in achieving the combination of desirable attributes present in certain of these methodologies which are not present in others.

In accordance with the present invention it has been recognized that simplified compositions and methods for detection of amplified nucleic acid sequences are required to fully exploit the documented utility of the amplification of specific DNA sequences. In accordance with the present invention, the following criteria have been fulfilled. It is sufficiently sensitive so that the rounds of amplification are kept to a minimum. It is suitably specific so that any nonspecific amplification is not erroneously scored as positive. It is adaptable to high volume and clinical testing applications, most preferably being readily adaptable to automation.

Further advantages of certain embodiments are that they provide methodologies for estimation of the efficiency of amplification so that negative results for the sequences of interest are scored as negative with increased confidence. Such systems also readily allow simplified methods for the amplification and detection of a plurality of target nucleic acid sequences within one sample and in a single assay operation. Additional advantages of such methods are that they provide simple, non-hazardous methodologies for partial purification of the target nucleic acids sequences of interest prior to initiation of the amplification cycles.

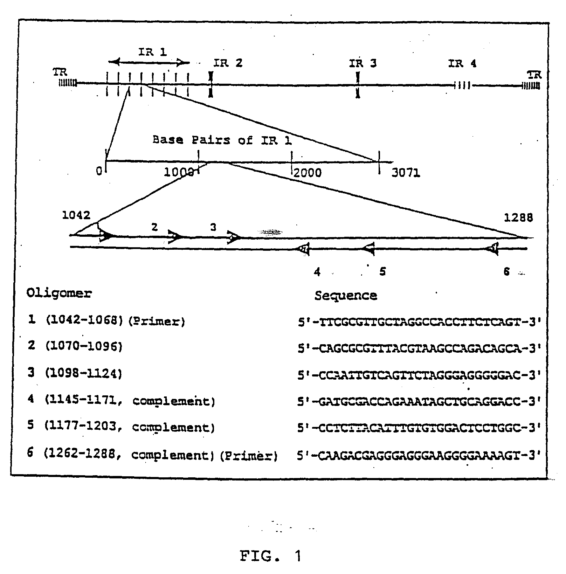

Fig. 1 is an illustration of the Epstein-Barr virus DNA structure and the oligonucleotide sequences used for amplification/hybrid capture assays. The IR1 repeat is enlarged to show the location of the oligonucleotide primers and probes. Listed in the lower portion of the figure are the sequences of oligonucleotides 1-6.

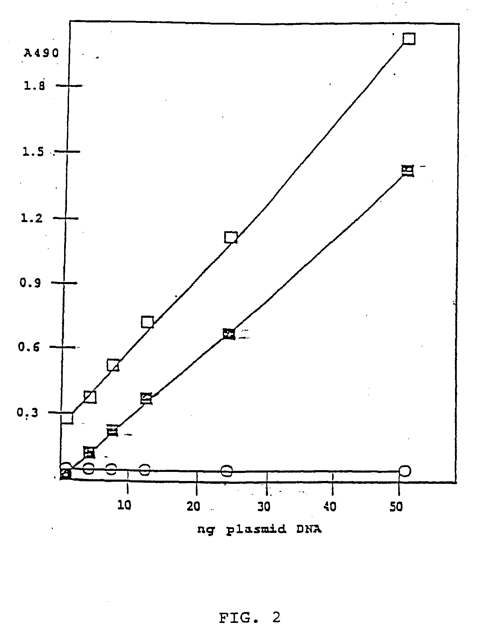

Fig. 2 is a graph which illustrates the sensitivity and specificity of the hybrid capture assay using unamplified nucleic acid target sequences. From 0.78 ng to 50 ng of the target plasmid DNA, pCF1, were assayed by hybrid capture with the oligonucleotide pairs, EBV 2 and poly T-tailed EBV 3 (open squares) or EBV 4 and poly T-tailed EBV 5(closed squares). Hybridization of equivalent amounts of pAK8 DNA with either of the oligonucleotide pairs generated background absorbance readings (open circles).

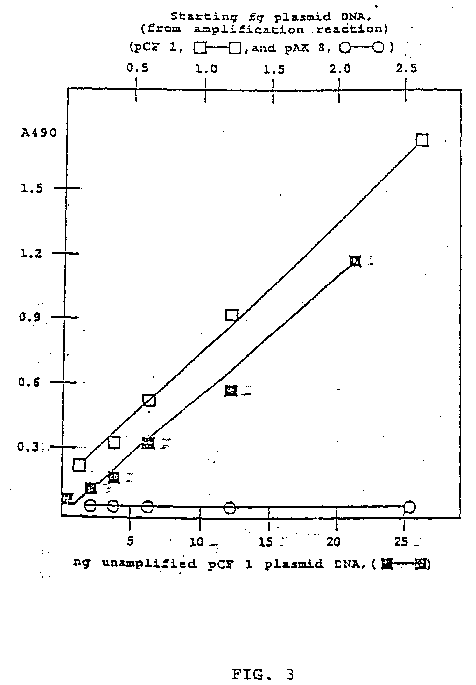

Fig. 3 is a graph which illustrates the detection of amplified EBV sequences by hybrid capture. One pg of pCF1 and pAK8 DNA were subjected to 25 rounds of amplification. Portions of the amplification reaction mixtures equivalent to 78 attograms to 2.5 femtograms of starting plasmid DNA were assayed by hybrid capture using EBV 5 as the capture oligonucleotide and poly T-tailed EBV 4 as the signal oligonucleotide. Unamplified pCF1 (0.625 ng - 20 ng) was assayed using the same oligonucleotide pair in order to generate a standard.

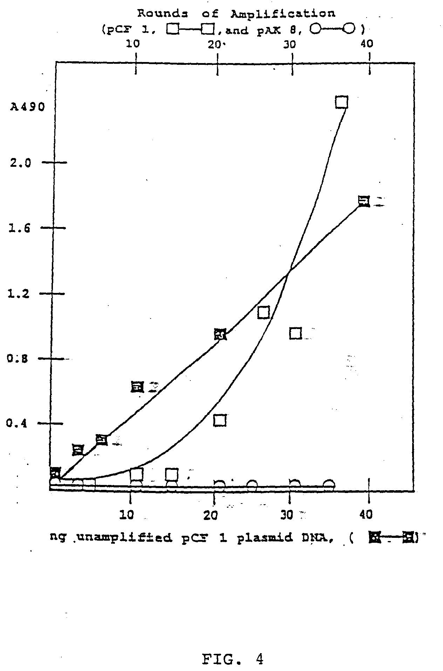

Fig. 4 is a graph which illustrates the relative increase in the amount of target DNA during the enzymatic amplification of nucleic acid sequences. Ten pg of pCF1 and pAK8 DNA were subjected to 0-35 rounds of amplification. At intervals of 5 rounds, amplificition reactions were terminated and asayed by hybrid capture using EBV 5 and poly T-tailed EBV 4. From 1.25 ng to 40 ng of unamplified pCF1 was also assayed by hybrid capture to generate a standard curve by which to estimate efficiencies of amplification.

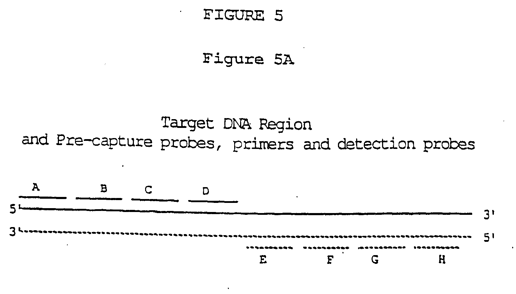

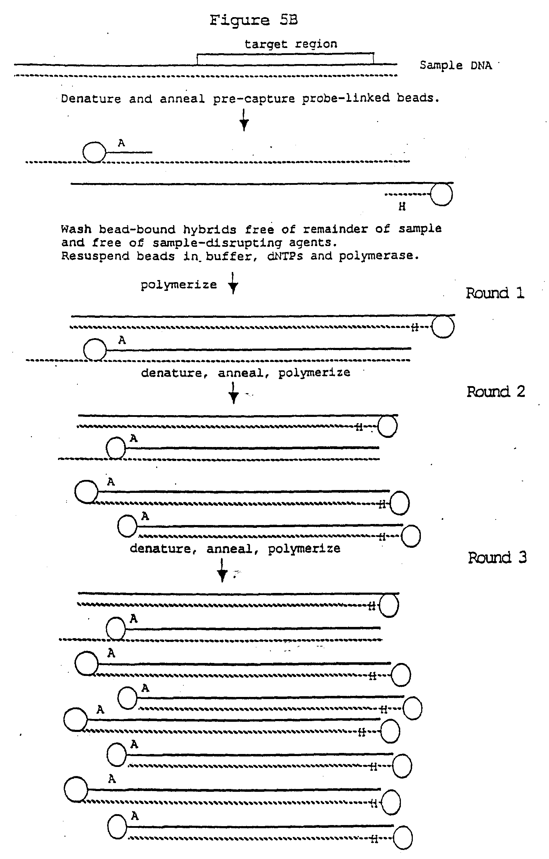

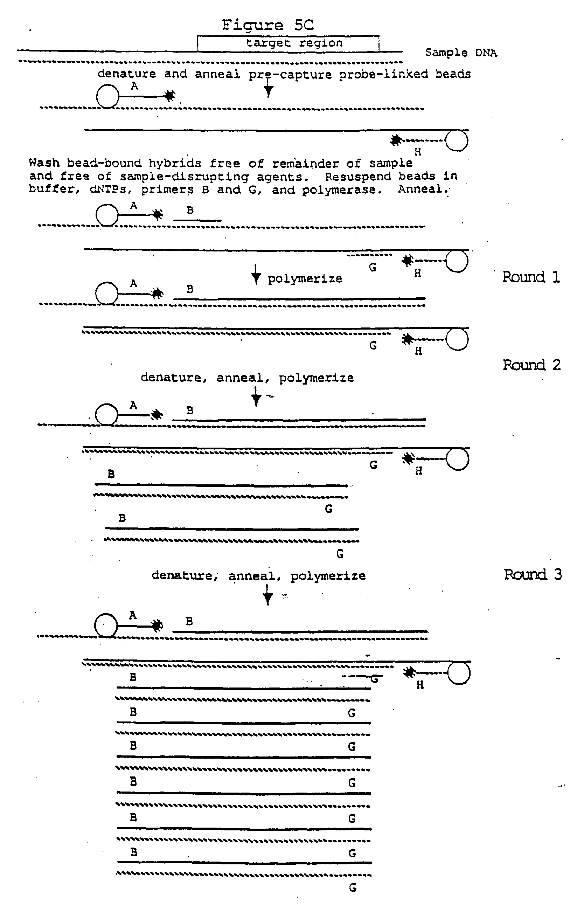

Fig. 5 is a diagram which illustrates pre-capture and amplification of target nucleic acid sequences. Fig. 5A illustrates the region of target nucleic acid in a DNA target. Pre-capture, primer and probe oligonucleotides are illustrated by the lines above and below the DNA duplex. A through D contain representative sequences that are the same sequences as those contained in the strand of DNA illustrated by a solid line. E through H contain representative sequences that are the same sequences as those contained in the strand of DNA illustrated by a hatched line. In Fig. 5B, pre-capture probes A and H are shown linked to beads as described in the text. They are hybridized to the target DNA strands. After washing of the bead-bound hybrids, amplification is carried out using the pre capture probes A and H (three rounds are illustrated), resulting in amplified DNA sequences linked to the bead-bound, pre-capture sequences A and H. Detection of the amplified nucleic acid sequences is carried out by hybridization and detection of bound signal probes B, C, D, E, F or G. In Fig. 5C bead-bound, pre-capture probes A and H are modified at the 3'termini, illustrated by the darkened ball at the end of the oligonucleotides. Following washing of the bead-bound hybrids of target nucleic acid and the pre-capture probes, amplification is carried out in the presence of primers B and G. Following amplification (3 rounds are illustrated) probe pairs C and D and/or E and F are used for detection of the amplified nucleic acid sequences that are not linked to the beads. In Figs. 5B and 5C, DNA synthesized during the polymerization steps of the rounds of amplification are shown as thickened solid or hatched lines.

As previously noted, the present invention provides compositions and methods for determining the presence or absence of nucleic acid analytes in a sample.

Sample fluids on which tests are performed include biological, physiological, industrial, environmental, and other types of liquids. Of particular interest are biological fluids such as serum, plasma, urine, cerebrospinal fluid, saliva, milk broth and other culture media and supernatants as well as mixtures or fractions of any of them. Physiological fluids of interest include infusion solutions, buffers, preservative or antimicrobial solutions and the like. Industrial liquids include fermentation media and other processing liquids used, for example, in the manufacture of pharmaceuticals, dairy products and malt beverages. Other sources of sample fluid which are tested by conventional methods are contemplated by this term as used, and can be assayed in accordance with the invention. Also contemplated for testing are tissue samples, including for example, hair, biopsy or tissue section samples, whether fresh, frozen or paraffin embedded.

The term "analyte" or "target" refers to any nucleic acid-containing substance whose presence is to be qualitatively or quantitatively determined in a sample fluid. The analyte usually is an oligo- or polynucleotide for which a complementary nucleic acid sequence exists or can be prepared. The analyte or target is usually an RNA or DNA.

The terms "matrix" or "test zone" refer to a carrier or substrate, such as a microscope slide, microtiter well, particle or the like which is insoluble in and maintains its structural integrity when exposed to test samples, water or physiological fluids and reagents used in their testing. Suitable materials include glass or organo-plastic materials, such as polystyrene, polypropylene or the like.

The terms "universal detection", "universal label-capturing moiety" and "universal label" refer to the labeling system described in the commonly assigned, co-pending application to Pergolizzi, et al., entitled Assay Method Utilizing Polynucleotide Sequences, which has U.S. serial no. 922,757 and was filed on October 24, 1986. The disclosure of this application is incorporated herein by reference.

Amplifying the copy number as described above can be, for example, by polymerase chain reaction or otherwise reproducing the genotypically specific portion of the nucleic acid material (e.g., transcription-based amplification).

Examples of the first and second entities, referred to below, include those wherein: one entity is a ligand and the other entity is a receptor therefor; the first and second entities are complementary polynucleotide homopolymers; one entity is an antigen or hapten and the other entity is an antibody or specific binding fragment thereof for the antigen or hapten; one entity is a hormone and the other entity is a receptor therefor; one entity is an enzyme and the other entity is an inhibitor thereof; one entity is an apoenzyme and the other entity is a cofactor therefor; one entity is a sugar and the other entity is a lectin which is specifically bindable therewith; and one entity is avidin or streptavidin and the other entity is biotin or a binding analog thereof.

In one aspect, the invention provides a composition for the determination of single-stranded target nucleic acids in a sample to be tested. The composition comprises (i) an oligonucleotide primer for each single-stranded target nucleic acid of interest, which primer is capable of annealing selectively to a complementary portion of the single strand of target nucleic acid; (ii) mononucleotides of each of the four nucleic acid bases; (iii) nucleic acid polymerases capable of producing an extension product from the primer and mononucleotides which is complementary to the single strand of target nucleic acid to which the primer is annealed to provide amplified nucleic acid sequences; (iv) a first polynucleotide probe comprising a universal label-capturing moiety attached to a first single-stranded polynucleotide segment which is hybridizable with a first portion of the target nucleic acid; (v) a second polynucleotide probe comprising a second single-stranded polynucleotide segment hybridizable with a second portion of the target nucleic acid; (vi) a matrix to which the second polynucleotide probe is affixed; and (vii) a universal label capturable by the first universal label-capturing moiety when the first polynucleotide probe is captured by hybridization to an amplified target nucleic acid sequence which is captured by hybridization with the matrix-affixed second polynucleotide probe.

Further related to this aspect, the present invention provides a method for the determination of single-stranded target nucleic acid in a sample to be tested. The method comprises (a) amplifying the copy number of the target nucleic acid; (b) contacting, under conditions permissive of hybridization, the amplified copy number of the target nucleic acid with a first polynucleotide probe comprising a first universal label-capturing moiety attached to a first single-stranded polynucleotide segment, the first single-stranded polynucleotide segment being hybridizable with a first portion of the target nucleic acid so as to form a hybrid; (c) contacting, under conditions permissive of hybridization, any hybrid soformed with a matrix-affixed second polynucleotide probe comprising a second single-stranded polynucleotide segment hybridizable with a second portion of the target nucleic acid so as to form a bound complex; and (d) separating, if necessary, the bound complex from any unbound nucleic acid; and (e) determining the presence or absence of the amplified target nucleic acid by capturing and observing the presence or absence of the universal label.

In a second aspect, the invention provides a composition for the determination of single-stranded target nucleic acids in a sample to be tested. This composition comprises (i) an oligonucleotide primer for each single-stranded target nucleic acid of interest, which primer is capable of annealing selectively to a complementary portion of the single strands of target nucleic acid; (ii) mononucleotides of each of the four nucleic acid bases; (iii) nucleic acid polymerases capable of producing an extension product from the primer and mononucleotides which is complementary to the single strand of target nucleic acid to which the primer is annealed; (iv) a first polynucleotide probe comprising at least one component of a labeling system attached to a first single-stranded polynucleotide segment which is hybridizable with a first portion of the target nucleic acid; (v) a second polynucleotide probe comprising a first entity attached to a second single-stranded polynucleotide segment which is hybridizable with a second portion of the target nucleic acid; and (vi) a matrix having attached thereto a second entity which is specifically bindable with the first entity.

Further related to this aspect, the present invention provides a method for the determination of single-stranded target nucleic acid in a sample to be tested. This method comprises (a) amplifying the copy number of the target nucleic acid; (b) contacting, under conditions permissive of hybridization, the amplified copy number of the target nucleic acid with (i) a first polynucleotide probe comprising at least one component of a labeling system attached to a first single-stranded polynucleotide segment which is hybridizable with a first portion of the target nucleic acid, (ii) a second polynucleotide probe comprising a first entity attached to a second single-stranded polynucleotide segment which is hybridizable with a second portion of the target nucleic acid, and (iii) a matrix having attached thereto a second entity which is specifically bindable with the first entity; (c) permitting hybridization of the first and second probes with any of the amplified copy number of the target nucleic acid present and binding of the first entity with the second entity to produce a bound complex; (d) separating, if necessary, the bound complex from any unbound nucleic acid; and (e) determining the presence or absence of the amplified target nucleic acid.

Another aspect of the invention provides for a pre-amplification capture step for improving the efficiency of amplification and detection of single-stranded target nucleic acid and reagents therefor. One embodiment provides a composition which comprises (a) a matrix-attached capture polynucleotide for each single-stranded target nucleic acid of interest, which polynucleotide is capable of annealing selectively to a complementary portion of its target nucleic acid; as well as serving as a primer for its target nucleic acid; (b) mononucleotides of each of the four nucleic acid bases; (c) nucleic acid polymerases capable of producing an extension product from the primer and mononucleotides which is complementary to the single strand of target nucleic acid to which the primer is annealed to provide matrix-bound amplified nucleic acid sequences; (d) a polynucleotide probe comprising at least one component of a labeling system attached to a single-stranded polynucleotide segment which is hybridizable with a portion of the amplified, matrix- bound target nucleic acid.

Another embodiment of this aspect provides a method which comprises (a) contacting so as to permit hybrid formation between the single-stranded target nucleic acid and a matrix-affixed capture polynucleotide for each single-stranded target nucleic acid of interest; (b) separating the hybridized target nucleic acid from the sample; (c) amplifying the copy number of the target nucleic acid to generate matrix bound amplified copy numbers of target nucleic acid; (d) contacting, under conditions permissive of hybridization, the amplified copy numbers with a polynucleotide probe comprising at least one component of a labeling system attached to a single-stranded polynucleotide segment which is hybridizable with a portion of the amplified target nucleic acid; (e) permitting hybridization to form a complex between amplified target nucleic acid and probe; (f) separating, if necessary, the bound probe from any unbound nucleic acid; and (g) determining the presence or absence of the bound amplified target by observing the bound label.

Another embodiment of this aspect is a composition which comprises (a) a matrix-attached capture polynucleotide for each single-stranded target nucleic acid of interest, which polynucleotide is capable of annealing selectively to a complementary portion of its target nucleic acid; (b) an oligonucleotide primer for each single-stranded target nucleic acid of interest, which primer is capable of annealing selectively to a complementary portion of its target nucleic acid; (c) mononucleotides of each of the four nucleic acid bases; (d) nucleic acid polymerases capable of producing an extension product from the primer and mononucleotides which is complementary to the single strand of target nucleic acid to which the primer is annealed; (e) a first polynucleotide probe comprising at least one component of a labeling system attached to a first single-stranded polynucleotide segment which is hybridizable with a first portion of the target nucleic acid; (f) a second polynucleotide probe comprising a first entity attached to a second single-stranded polynucleotide segment which is hybridizable with a second portion of the target nucleic acid; and (g) a matrix having attached thereto a second entity which is specifically bindable with the first entity.

Another embodiment of this aspect is a method which comprises (a) contacting the single-stranded target nucleic acid, under conditions permissive of hybridization, with at least one matrix-attached capture polynucleotide for each single-stranded target nucleic acid of interest, which polynucleotide is capable of annealing selectively to a complementary portion of its target nucleic acid; (b) separating the hybridized target nucleic acid from the sample; (c) amplifying the copy number of the target nucleic acid by adding primer non-overlapping with capture sequences to generate unbound amplified copy numbers of target nucleic acid; (d) contacting, so as to form a complex, the unbound amplified target nucleic acid with (i) a first polynucleotide probe comprising at least one component of a labeling system attached to a first single-stranded polynucleotide segment which is hybridizable with a first portion of the target nucleic acid (ii) a second polynucleotide probe comprising a first entity attached to a second single-stranded polynucleotide segment hybridizable with a second portion of the target nucleic acid; (e) contacting the complex with a matrix having attached thereto a second entity which is specifically bindable with the first entity; (f) separating, if necessary, the bound complex from any unbound nucleic acid; and (g) determining the presence or absence of the bound amplified target nucleic acid by observing the presence of bound label.

In another aspect, the invention provides compositions for determining single-stranded target nucleic acid and for confirming the efficiency of amplification thereof in a sample containing a marker nucleic acid and suspected of containing target nucleic acid. In one embodiment the composition comprises (a) an oligonucleotide primer for each single-stranded target nucleic acid in a reaction zone, which primer is capable of annealing selectively to a complementary portion of the single-stranded target nucleic acid; (b) an oligonucleotide primer for each single-stranded marker nucleic acid, whose copy number in the sample is known, in the reaction zone, which primer is capable of annealing selectively to a complementary portion of the single-stranded marker nucleic acid; (c) mononucleotides of each of the four nucleic acid bases; (d) nucleic acid polymerases capable of producing in the reaction zone extension products from the primers and mononucleotides which are complementary to the single strand of target and marker nucleic acids, respectively, to which the primers are annealed; (e) a first polynucleotide target probe comprising at least one component of a labeling system attached to a first single-stranded polynucleotide segment hybridizable with a first portion of the target nucleic acid; (f) a second polynucleotide target probe bound to a first test zone separate from the reaction zone and comprising a second single-stranded polynucleotide segment hybridizable with a second portion of the target nucleic acid; (g) a first polynucleotide marker probe comprising at least one component of a second labeling system attached to a first single-stranded polynucleotide segment which is hybridizable with a first portion of the marker nucleic acid; and (h) a second polynucleotide marker probe bound to a second test zone separate from the reaction zone and the first test zone and comprising a second single-stranded polynucleotide segment hybridizable with a second portion of the marker nucleic acid.

Another embodiment of this aspect is a method which comprises (a) contacting the sample in a reaction zone with an oligonucleotide primer capable of annealing selectively to a complementary portion of the single-stranded target nucleic acid; (b) contacting the sample in the reaction zone containing a known copy number of marker nucleic acid with an oligonucleotide primer capable of annealing selectively to a complementary portion of the single-stranded marker nucleic acid; (c) amplifying the copy number of the target and marker nucleic acids in the reaction zone by contact with a nucleic acid polymerase; (d) contacting in the reaction zone, under conditions permissive of hybridization, the amplified copy number of target and marker nucleic acid with (i) a first polynucleotide target probe, comprising at least one component of a first labeling system attached to a first single-stranded polynucleotide segment hybridizable with a first portion of the target nucleic acid and (ii) a first polynucleotide marker probe, comprising at least one component of a second labeling system attached to a first single-stranded polynucleotide segment hybridizable with a first portion of the marker nucleic acid; (e) permitting hybridization of the first marker and target probes with the amplified copy number of marker nucleic acid and any target nucleic acid which may be present, respectively, so as to form marker and target hybrids; (f) contacting a first aliquot of the sample so-treated in a first test zone separate from the reaction zone with a second polynucleotide target probe bound to the first test zone so as to permit hybridization of the second target probe with any of the amplified copy number of target nucleic acid present; (g) contacting a second aliquot of the sample so-treated in a second test zone separate from the reaction zone and the first test zone with a second polynucleotide marker probe bound to the second test zone so as to permit hybridization of the second marker probe with the amplified copy number of marker nucleic acid; (h) separating, if necessary, from the test zones any unbound nucleic acid; and (i) determining the copy number of any target nucleic acid present in the first test zone and the copy number of marker nucleic acid in the second test zone. In the above embodiments, the first and second labeling systems can be the same or different.

Another embodiment of this aspect is a composition which comprises (a) an oligonucleotide primer for each single-stranded target nucleic acid in a reaction zone, which primer is capable of annealing selectively to a complementary portion of the single-stranded target nucleic acid; (b) an oligonucleotide primer for each single-stranded nucleic acid whose copy number in the sample is known, in the reaction zone which primer is capable of annealing selectively to a complementary portion of the single-stranded marker nucleic acid; (c) mononucleotides of each of the four nucleic acid bases; (d) nucleic acid polymerases capable of producing in the reaction zone extension products from the primers and mononucleotides which are complementary to the single strands of target and marker nucleic acids, respectively, to which the primers are annealed; (e) a first polynucleotide target probe comprising at least one component of a first labeling system attached to a first single-stranded polynucleotide segment hybridizable with a first portion of the target nucleic acid; (f) a second polynucleotide target probe comprising a first entity attached to a second single-stranded polynucleotide segment hybridizable with a second portion of the target nucleic acid; (g) a target test zone having bound thereto a second entity which is specifically bindable with the first entity; (h) a first polynucleotide marker probe comprising at least one component of a second labeling system attached to a first single-stranded polynucleotide segment hybridizable with a first portion of the marker nucleic acid; (i) a second polynucleotide marker probe comprising a third entity attached to a second single-stranded polynucleotide segment hybridizable with a second portion of the marker nucleic acid; and (j) a target test zone having bound thereto a fourth entity which is specifically bindable with the third entity.

In the above embodiment, the first and second labeling systems can be the same where the first and second binding entity pair is different from the third and forth binding entity pair. Alternatively, where the first and second labeling systems are distinguishably different, the first and second binding entity pair can be the same as the third and forth binding entity pair.

Another embodiment of this aspect is a method which comprises (a) contacting the sample with an oligonucleotide primer for each single-stranded target nucleic acid in a reaction zone, which primer is capable of annealing selectively to a complementary portion of the single-stranded target nucleic acid; (b) contacting the sample with an oligonucleotide primer for each single-stranded marker nucleic acid in the reaction zone,the copy number of which in the sample is known, which primer is capable of annealing selectively to a complementary portion of the single-stranded marker nucleic acid; (c) amplifying in the reaction zone the copy number of the target and marker nucleic acids in the sample by contacting the sample with a nucleic acid polymerase; (d) contacting in the reaction zone, under conditions permissive of hybridization, the amplified copy number of target nucleic acid with (i) a first polynucleotide target probe comprising a label moiety and a first single-stranded polynucleotide segment hybridizable with a first portion of the target nucleic acid attached to the label moiety and (ii) a second polynucleotide target probe comprising a second single-stranded polynucleotide segment hybridizable with a second portion of the target nucleic acid and a first entity attached to the second polynucleotide segment; (e) contacting in the reaction zone, under conditions permissive of hybridization, the amplified copy number of marker nucleic acid with (i) a first polynucleotide marker probe comprising a label moiety and a first single-stranded polynucleotide segment hybridizable with a first portion of the marker nucleic acid attached to the label and (ii) a second polynucleotide marker probe comprising a second single-stranded polynucleotide segment hybridizable with a second portion of the marker nucleic acid and a third entity attached to the second polynucleotide segment; (f) permitting hybridization of (i) the first and second target probes with any of the amplified copy number of target nucleic acid present and (ii) the first and second marker probes with the amplified copy number of marker nucleic acid present; (g) contacting an aliquot of the sample as treated in steps (a)-(f) with a second entity specifically bindable with the first entity in a first test zone which is separate from the reaction zone and to which the second entity is bound; (h) contacting another aliquot of the sample as treated in steps (a)-(f) with a fourth entity specifically bindable with the third entity in a second test zone which is separate from the reaction zone and first test zone and to which the fourth entity is bound; (i) separating nucleic acid bound to a test zone from any unbound nucleic acid; and (j) determining the copy number of the amplified marker nucleic acid and the presence or absence of any target nucleic acid.

In yet another aspect, the present invention provides a composition for the amplification and detection of a plurality of single-stranded target nucleic acid sequences in a sample to be tested. Multiple sets of polynucleotide primers allow the specific amplification of more than one target nucleic acid sequence within a single amplification reaction mixture and the use of multiple sets of first and second polynucleotide probes allows for the detection of more than one species of amplified target nucleic acid sequence. These compositions are useful for simplifying the determination of efficiency of an amplification reaction in order to establish reliability of negative results. They are also useful for performing more than one determination at a time on a particular sample to be tested.

In one embodiment, the invention provides a composition for the determination of a plurality of single-stranded target nucleic acids in a sample to be tested. This composition comprises (a) an oligonucleotide primer for each single-stranded target nucleic acid of interest, each of which primers is capable of annealing selectively to a complementary portion of one of the single strands of target nucleic acid; (b) mononucleotides of each of the four nucleic acid bases; (c) nucleic acid polymerases capable of producing in a reaction zone extension products from the primers and mononucleotides, which extension products are complementary to the single strand of target nucleic acid to which a particular primer is annealed; (d) a first polynucleotide probe for each of the nucleic acid targets comprising a label moiety and a first single-stranded polynucleotide segment attached to the label moiety, each of the first single-stranded polynucleotide segment being hybridizable with a first portion of its respective target nucleic acid; (e) a second polynucleotide probe for each of the nucleic acid targets comprising a second single-stranded polynucleotide segment hybridizable with a second portion of its target nucleic acid and being bound to a test zone separate from each of the other target nucleic acid test zones.

In another embodiment of this aspect, the invention provides a composition for determining a plurality of single-stranded target nucleic acids in a sample to be tested. This composition comprises (a) an oligonucleotide primer for each single-stranded target nucleic acid of interest, each of which primers is capable of annealing selectively to a complementary portion of one of the single strands of target nucleic acid; (b) mononucleotides of each of the four nucleic acid bases; (c) nucleic acid polymerases capable of producing in a reaction zone extension products from the primers and mononucleotides, which extension products are complementary to the single strand of target nucleic acid to which a particular primer is annealed; (d) a first polynucleotide probe for each of the nucleic acid targets comprising a label attached to a first single-stranded polynucleotide segment hybridizable with a first portion of its target nucleic acid; (e) a second polynucleotide probe for each of the nucleic acid targets comprising a second single-stranded polynucleotide segment hybridizable with a second portion of its target nucleic acid and a specific binding entity attached to the second polynucleotide segment; and (f) a plurality of test zones each separate from the reaction zone and each of the other test zones and each having attached thereto a binding partner that will bind only with the specific binding entity attached to the second probe for the target nucleic acid of interest.

In another aspect the invention provides a composition and method that combine the attributes of amplification with those of hybridization agglutination. Hybridization agglutination is described in detail in the commonly assigned, co-pending application to Engelhardt and Rabbani, entitled Hybridization Method for the Detection of Genetic Material which was filed on January 15, 1987 as U.S. serial no. 005,327. The disclosure of this application is incorporated herein by reference. This application is derived from U.S. serial no. 653,816 (filed September 24, 1984), which is derived from U.S. serial no. 605,022 (filed April 27, 1984).

One embodiment of this aspect is a composition which comprises (a) an oligonucleotide primer for each single-stranded target nucleic acid of interest, which primer is capable of annealing selectively to a complementary portion of the single strand of target nucleic acid; (b) mononucleotides of each of the four nucleic acid bases; (c) nucleic acid polymerases capable of producing an extension product from the primer and mononucleotides which is complementary to the single strand of target nucleic acid to which the primer is annealed to provide amplified nucleic acid sequences; (d) a first polynucleotide probe comprising a first particle to which is attached a plurality of first single-stranded polynucleotide segments complementary to a first portion of the target nucleic acid; and (e) a second polynucleotide probe comprising a second particle having attached thereto a plurality of second single-stranded polynucleotide segments complementary to a second portion of the target nucleic acid.

Another embodiment of this aspect provides a which method the steps of (a) amplifying the copy number of the target nucleic acid; (b) contacting, under conditions permissive of hybridization, the target nucleic acid with (i) a first polynucleotide probe comprising a first particle to which is attached a plurality of first single-stranded polynucleotide segments complementary to a first portion of the target nucleic acid, and (ii) a second polynucleotide probe comprising a second particle having attached thereto a plurality of second single-stranded polynucleotide segments complementary to a second portion of the target nucleic acid; (c) permitting hybridization of the first polynucleotide segments to the first portion of target nucleic acid and the second segments to the second portion of target nucleic acid the hybridizations resulting in agglutination; and (d) detecting the agglutination.

### Example 1

Epstein-Barr virus (EBV) sequences contained in purified preparations of plasmid DNA as well as in crude cell lysates of EBV-infected lymphoid cell cultures were enzymatically amplified by the polymerase chain reaction technique and detected by hybrid capture.

### Reagents

Chemical reagents were purchased from Aldrich Co. unless otherwise stated. Detek 1-hrp, Detek-1 alk, BioBridge A, terminal deoxynucleotide transferase and biotin-11-dUTP were from Enzo Biochem, Inc. Thermus aquaticus (Tag) polymerase was purchased from Perkin-Elmer/Cetus. pUC18 was obtained from International Biotechnologies, Inc. Deoxynucleoside triphosphates and dextran sulfate were obtained from Pharmacia. Ortho-phenylenediamine dihydrochloride (OPD), porcine skin gelatin, hydrogen peroxide, Sephadex G-25 and Triton X-100 were obtained from Sigma. Immulon 2 microtiter plates were purchased from Dynatech. Formamide was obtained from Fluka. RPMI 1640 culture medium and heat inactivated fetal bovine serum were purchased from Gibco. Biodyne B membranes were purchased from Pall Corporation. NuSieve agarose and Seakem agarose were purchased from FMC.

The buffers used in the hybridization and detection assays were prepared from the following stock solutions: 1) 20X SSC containing 3M NaCl, 0.3M sodium citrate; 2) 20X SSPE containing 3.6M NaCl, 0.2M sodium phosphate buffer, pH 7.4, and 0.02M ethylenediamine tetraacetic acid (EDTA); 3) Citrate phosphate buffer, pH 5.3, containing 0.05M citric acid and 0.1M Na₂HPO₄ combined to give a pH of 5.3; 4) lOX PBS containing 1.5M NaCl, 0.1M sodium phosphate buffer, pH 7.2; and 5) Denhardt's solution containing 1% Ficoll, 1% polyvinylpyrrolidone and 1% bovine serum albumin.

### Oligonucleotide Synthesis, Purification and Labeling

Oligonucleotides were synthesized on an Applied Biosystems, Inc. instrument, model 380 B, by the phosphoramidite method. Both 0.2 umol and 1 umol synthesis cycles were employed. The cyanoethyl phosphoramidites and reagents were used as recommended by the manufacturer. Phosphoramidite additions during chain elongation were monitored by release of trityl cation. A procedure which left the 5'-hydroxyl protected as a dimethoxytrityl ether after the oligonucleotide was cleaved from the support was used. Following synthesis and cleavage from the support, the oligomers were deprotected by treatment with ammonia at 55°C overnight and the solutions were subsequently evaporated to dryness.

5'-0-dimethoxytrityl oligonucleotides were redissolved in 1 ml of 10 mM triethylammonium bicarbonate, pH 7.5-9.0, and were purified using a Whatman Partisil M-20 ODS-3 (C₁₈, semi-preparative) HPLC column. The column was equilibrated in acetonitrile:0.1 M triethylammonium acetate, pH 7.0 (TEAA) at 1:99. Up to 75 absorbance units (260 nm) were injected onto the column. The oligonucleotides were eluted at a flow rate of 4 ml/minute using 1:99 acetonitrile:TEAA for 1 minute followed by a linear gradient of 1 to 30% acetonitrile over 20 minutes. The tritylated oligomers eluted as single peaks at retention times ranging from 19 to 23 minutes. After evaporation of solvent, oligomers were detritylated by dissolution in 0.5 ml 80% acetic acid for 20 minutes and subsequently lyophilized. Final purification was achieved by Sephadex G-25 chromatography in 10 mM ammonium acetate. Fractions containing oligomers were lyophilized and resuspended in sterile water.

Oligonucleotides were 3'-terminally labeled with approximately 3-4 biotin-11-dUTP residues per molecule as previously described by Cook et al., Synthesis and Hybridization of a Series of Biotinylated Oligonucleotides, Nucleic Acids Research, 16:4077-4095 (1988). Signal oligonucleotides for hybrid capture assays were 3'-terminally labeled with a polymer of TMP in reaction mixtures containing 0.2M potassium cacodylate buffer, pH 7.2, 1 mM 2-mercaptoethanol, terminal transferase (20-50 units/ug of oligomer), 0.4 mM TTP and 20 ug/ml oligonucleotide. Reactions were allowed to go to completion (90 minutes to 2 hours at 37°C) and were terminated by the addition of EDTA to a final concentration of 25 mM. Biotin-labeled and poly T-labeled oligonucleotides were used without additional purification.

### Preparation of Cell Lysates

B95-8, Raji, Namalwa and Ramos cells were maintained in RPMI 1640 media containing 10% heat inactivated fetal calf serum. Cells were collected by low speed centrifugation, medium was removed, and the cells were resuspended in 10 ul of PBS. The sample volume was increased to 45 ul with sterile water and subjected to enzymatic amplification.

### Amplification Reactions

Purified plasmid DNA or lymphoid cell lysates in a 45 ul volume were heated at 92°C for 10 minutes and quick chilled. The boiled samples were mixed with 55 ul of a 1.8X stock of reaction buffer such that the final reaction mixture contained 50 mM KCl, 10 mM Tris-HCl (pH 8.0), 1.5 mM MgCl₂, 0.01% gelatin, 200 uM each of dATP, dGTP, dCTP and dTTP and 1 uM of each oligonucleotide primer. The reaction was initiated by the addition of 2.5 Units of Tag polymerase. The samples were repeatedly cycled through a 1 minute denaturation step at 92°C , a 2 minute annealing step at 37°C and a 3 minute extension step at 70°C. Following the final cycle, the samples were heated at 92°C for 1 minute and quick chilled.

### Hybrid Capture and Detection

Individual wells of 96 well Immulon 2 plates were rinsed 2 times with 1 M ammonium acetate. Fifty ng of capture oligonucleotide were added to the wells of the plates in 1 M ammonium acetate. Plates were incubated at 37°C overnight to dry and were then either used immediately or were stored at 4°C prior to use. Plates with bound capture oligonucleotide were pre-washed with 0.5X SSC, 0.1% Triton X-100 in preparation for use in the hybrid capture assay.

Samples to be analyzed were heat denatured at 92°C for 10 minutes, quick chilled and diluted in sterile water. The diluted samples were added to hybridization solutions containing final concentrations of 20% formamide, 1X SSPE, 2% dextran sulfate, 1% Triton X-100 and 50 ng/ml of the signal oligonucleotide containing a 3'-poly T tail. One hundred microliters of each sample were added in triplicate to oligonucleotide coated wells and hybridized (with shaking) for 1 hour at room temperature. Following hybridization, the wells were washed 4 times with 0.05X SSC, 0.1% Triton X-100 and BioBridge A (diluted to Enzo's specifications in 2X SSC, 0.1% Triton X-100) was added to each well and incubated for 2.5 minutes at room temperature. The wells were washed 3 times with 0.5X SSC, 0.1% Triton X-100 and Detek 1-hrp brand streptavidin horseradish peroxidase complex, diluted in phosphate-buffered saline containing 0.5M NaCl, 5mM EDTA, 0.3% gelatin, 0.25% Triton X-100, and 0.01% thimerosol was added to each well and incubated (with shaking) for 15 minutes at room temperature. The wells were washed 3 times with 0.5X SSC, 0.1% Triton X-100, followed by 3 washes with PBS containing 5 mM EDTA. The signal was generated by the addition of 150 ul of reaction mixture containing 0.0125% hydrogen peroxide and 1.6 mg/ml ortho-phenylenediamine in citrate phosphate buffer (pH 5.3). Color development was terminated by the addition of 50 ul of 4N H₂SO₄ and optical density measurements at 490 nm were made on an Intermed Immunoreader model NJ 2000. Other chromogenic indicators can also be used, such as 3, 3', 5, 5'-tetramethylbenzidene.

### Agarose Gel Electrophoresis and Southern Blot Hybridization

Amplified products were subjected to electrophoresis in agarose gels containing 3% NuSieve agarose, 1% Seakem agarose in 0.04 M Tris-acetate, 0.002 M EDTA (pH 8.0) and 0.2 ug/ml ethidium bromide. Following electrophoresis, the DNA was denatured in 0.4 N NaOH, 0.6 N NaCl and transferred overnight to a Biodyne B membrane. The membrane was prehybridized for 60 minutes at 55°C in buffer containing 6X SSC, 5X Denhardt's solution, 0.1% SDS and 100 ug/ml heat denatured salmon sperm DNA. The DNA was hybridized to the oligonucleotide EBV 5 (100 ng/ml)(see Fig. 1) containing a 3'-biotin-11-dUMP tail in the prehybridization buffer with 10% dextran sulfate at 55°C. Following hybridization, the membrane was washed with 3X SSC, 0.1% SDS for 10 minutes at room temperature and 3 times (10 minutes per wash) at 55°C in the same buffer. The membrane was rinsed in 100 mM Tris.HCl (pH 8.0), 100 mM NaCl, 3 mM MgCl₂ and 0.5% Tween 20 (solution A), followed by a 15 minute pre-block with PBS containing 0.5% casein. The membrane was incubated at room temperature for 15 minutes in Detek 1-alk diluted in 100 mM Tris-HCl (pH 9.5), 100 mM NaCl, 3 mM MgCl₂ and 0.05% Tween 20. After washing with solution A, detection buffer containing 100 mM Tris-HCl (pH 9.5), 10 mM MgCl₂, 100 mM NaCl, 75 mg/ml nitroblue tetrazolium-and 50 mg/ml 5-bromo-4-chloro-3-indolyl-phosphate was added. Color development was terminated after approximately 5 minutes by removing the filter from detection buffer and rinsing it with water.

### Detection of Epstein-Barr Virus DNA Sequences by Hybrid Capture

Epstein-Barr virus (EBV) sequences contained in purified plasmid DNA preparations as well as in crude cell lysates of EBV-infected lymphoid cell cultures were chosen as targets for amplification and detection. The IR1 repeat of EBV is reiterated approximately 10 times per EBV genome, as reported by Cheung and Kieff, Long internal direct repeat in Epstein-Barr virus DNA, J. Virology, 44:286-294 (1982). Six oligonucleotides spanning a 246 base pair sequence of the IR1 repeat were selected for use in the coupled amplfication/hybrid capture assays. They are shown in the lower portion of Fig. 1. To optimize the hybridization and detection of IR1 target sequences, several oligonucleotide pairs were tested in the hybridization assay for their ability to capture and signal the plasmid, pCF1, which contains the BamH1 cleavage fragment of the IR1 repeat in the pUC18 vector. In each instance, one (or two) oligonucleotide(s) was bound to the wells of an Immulon 2 plate and acted to capture the target sequence. A second oligonucleotide (or two oligonucleotides), 3'-terminally labeled with poly T, served as a link in the signaling reaction by coordinately hybridizing to the target sequence and to a biotin modified poly dA (BioBridge A brand reagent). Biotin was detected using a complex of streptavidin and biotinylated horseradish peroxidase in the presence of hydrogen peroxide and OPD. As a negative control, pAK8, which contains a 13 kb cytomegalovirus (CMV) DNA insert in pUC18, was assayed using the EBV-specific oligonucleotides. Standard curves were generated for known amounts of pCF1 hybridized with various combinations of the oligonucleotides shown in Fig. 1.

Fig. 2 shows the standard curves generated with 0.78 ng to 50 ng of pCF1 following hybridization with two different sets of oligonucleotide pairs. The limit of detection of the hybridization assay for unamplified pCF1 was approximately 5 X 10⁸ molecules of target plasmid DNA. Similar amounts of the CMV DNA-containing plasmid, pAK8, failed to generate a signal, thus demonstrating the specificity of the assay.

### Amplification and Detection of EBV Sequences in Purified Plasmid DNA

A 246 bp sequence bordered by the oligonucleotides EBV 1 and EBV 6 (Fig. 1) was enzymatically amplified by subjecting 1 pg of pCF1 to 25 rounds of PCR. Following amplification, portions of the amplification reaction mixture equivalent to 78 attograms to 2.5 femtograms of the starting plasmid DNA were assayed by hybrid capture using EBV 5 as the capture oligonucleotide and poly T-tailed EBV 4 as the signal oligonucleotide. Fig. 3 shows the absorbance at 490 nm of the amplified material compared to a standard curve generated by known amounts of unamplified pCF1. Following 25 rounds of amplification, the coupled PCR/hybrid capture assay was able to detect 12.5 to 400 molecules of target plasmid DNA. The estimated amplification of the target DNA sequence was approximately 1.2 X 10⁷ compared with a theoretical amplification limit of 3.3 X 10⁷ suggesting an efficiency of 92% (efficiency is calculated as Y = (1 + X)ⁿ where n = the number of rounds of amplification, X = the mean efficiency of each cycle and Y is the yield after n cycles. See Mullis and Faloona, Specific synthesis of DNA in vitro via a polymerase catalysed chain reaction, Methods Enzymol., 155:335-350 (1987). As a control, 1 pg of pAK8 DNA was amplified by 25 rounds of PCR in the presence of EBV 1 and EBV 6 and assayed by the same hybrid capture procedure. When as much as 5% (50 fg) of the original sample was assayed, no signal was generated. In similar studies, as much as 10 ng of control plasmid DNA, amplified and hybridized as above, failed to generate a signal. In addition, when either pCF1 or pAK8 were cycled through PCR in the absence of the Tag polymerase, no signal was generated in the hybridization assay.

Further referring to the sequences shown in Fig. 1, various primer combinations that work well with respective signal/capture oligonucleotide pairs are primers 1 and 6 with signal/capture oligonucleotide pairs 4 and 5 and/or 2 and 3, primers 2 and 6 with signal/capture oligonucleotide pairs 4 and 5, primers 3 and 6 with signal/capture oligonucleotide pair 4 and 5, primers 1 and 5 with signal/capture oligonucleotide pair 2 and 3, and primers 1 and 4 with signal/capture oligonucleotide pair 2 and 3.

To determine the relalive increase in the amount of target DNA throughout the amplification procedure, 10 pg of pCF1 and pAK8 were cycled through 0-35 rounds of PCR in the presence of the primers, EBV 1 and EBV 6. At intervals of 5 rounds, an amplification reaction was terminated and 0.03% of the reaction mixture was assayed. As demonstrated in Fig. 4, a signal was first detected after 20 rounds of amplification. This signal corresponded to a 1.7 X 10⁵-fold increase of target DNA suggesting an 80% efficiency of amplification. The amount of target DNA increased with successive rounds of amplification and at round 35, the signal generated by only 0.03% of the reaction mixture was beyond the linear absorbance range of the assay and the absolute level of amplification and the absolute level of amplification could not be accurately measured. The negative control, pAK8 DNA, treated similarly, did not generate a signal in the hybridization assay at any stage of the amplification procedure.

### Amplification and Detection of EBV Sequences in Crude Cell Lysates

As an example of the detection of target DNA contained in lysates of infected cells, the amplification and detection of EBV-specific sequences in crude cell lysates was examined. For these studies, four lymphoid cell lines were used. B95-8, an EBV-infected cell line in which 95% of the cells contain approximately 50 copies of EBV DNA and 5% of the cells contain greater than 1000 copies of EBV DNA, was used and is further described in Miller and Lipman, Release of infectious EBV by transformed marmoset leukocytes, Proc. Nat. Acad. Sci. USA, 70:190-194 (1973). Raji, a cell line in which all cells harbor approximately 50 copies of EBV DNA, was used and is further described in Epstein et al, Morphological and virological investigations on cultured Burkitt tumor lymphoblasts (strain Raji),J. Natl. Cancer Inst., 37:547-559 (1966). Namalwa, a cell line in which all cells contain 1-2 copies of EBV DNA, was used and is further described in Reedmen and Klein, Cellular localization of an EBV-associated complement-fixing antigen in producer and nonproducer lymphoblastoid cell lines, Int. J. Cancer, 11:449-520 (1973). Ramos, an uninfected lymphoid cell line, was also used and is further described in Klein et al., An EBV-genome-negative cell line established from an American Burkitt lymphoma: receptor characteristics, EBV infectibility and permanent conversion into EBV-positive sublines by in vitro infection, Intervirology, 5:319-334 (1975). From 10⁴ to 10⁶ cells of cach culture were lysed by boiling and were then subjected to 15 rounds of amplification in the presence of EBV 1 and EBV 6. A portion of each reaction mixture was then assayed by hybrid capture. Capture wells that had been hybridized (and detected) without target DNA were used as zero (blank) controls for the absorbance readings. The results are shown in Table 1.

**Table 1**

| Amplification/Detection in Crude Cell Lysates | | | |
|---|---|---|---|
| Cell Line | # of cells in PCR Rxn. | # of cells Assayed/Well | A490 |
| | | | |
| B95-8 | 10⁶ | 10⁴ | 2.536 |
| | 10⁴ | 10² | 1.865 |
| | | | |
| Raji | 10⁶ | 10⁴ | 0.424 |
| | 10⁴ | 10² | 0.563 |
| | | | |
| Namalwa | 10⁶ | 10⁴ | 0.204 |
| | 10⁴ | 10² | <0.00 |
| | | | |
| Ramos | 10⁶ | 10⁴ | <0.00 |
| | 10⁴ | 10² | <0.00 |
| | | | |

As shown in Table 1, signal was obtained with B95-8, Raji and Namalwa cells, but not with the Ramos cells. These data suggest that the specificity of the coupled amplification/hybrid capture assay was maintained even when the target was not purified DNA.

Because Namalwa cells contain 1 to 2 copies of EBV DNA, these cells were chosen to examine the sensitivity of the coupled assays. The results are shown in Table 2.

**Table 2**

| Sensitivity of Amplification/ Detection in Lysates of homogeneous Cell Populations | | | | |
|---|---|---|---|---|
| Cells | # of Cells in PCR Rxn. | # of Cells Assayed/Well | # of Rounds of PCR | A490 |
| | | | | |
| Namalwa | 10⁵ | 1250 | 25 | 1.36 |
| | 10⁴ | 50 | 30 | 1.63 |
| | 10³ | 50 | 35 | 1.47 |
| | 10² | 20 | 35 | 1.31 |
| | 10¹ | 2 | 35 | 0.15 |
| | | | | |
| Ramos | 10⁵ | 500 | 30 | 0.03 |
| | 10³ | 50 | 35 | 0.03 |
| | 10² | 20 | 35 | 0.01 |
| | 10¹ | 2 | 35 | 0.09 |
| | | | | |

As shown in Table 2, amplification of the 246 bp IR1 sequence in as few as 10 Namalwa cells generated a weak signal when 20% (equivalent of two cells) of the reaction mixture was assayed. The intensity of the signal dramatically increased when 100 cells were input into the initial amplification reaction. Amplification of an equivalent number of Ramos cells did not generate signal above background readings.

As an example of the detection of the nucleic acids of infectious agents within a heterogeneous population of infected and uninfected cells, Ramos and Namalwa cells were mixed, amplified by PCR, and assayed by hybrid capture. Namalwa cells (10²) were mixed with Ramos cells (10⁴) and were cycled through 35 rounds of PCR. A portion (20%) of the reaction mixture was then assayed. The results are shown in Table 3.

**Table 3**

| Sensitivily of Amplification/Detection in Lysates of Mixed Cell Populations | | | | |
|---|---|---|---|---|
| Cells | # of Cells in PCR Rxn. | # of Cells Assayed/Well | # of Rounds of PCR | A490 |
| | | | | |
| Namalwa | 10² | 20 | 35 | 0.88 |
| Ramos | 10⁴ | 2000 | 35 | 0.01 |
| Namalwa/ Ramos | 10²/10⁴ | 2020 | 35 | 0.24 |
| | | | | |

As shown in Table 3, the target sequence was amplified and detected even in the mixed cell lysates. The level of amplification in the cell mixture was diminished approximately 4-fold when compared to the homogeneous cell population. Since amplification of Ramos cells alone did not generate signal in the hybridization assay, it can be assumed that the signal generated by the mixed lysates was the product of the specific target sequence in the Namalwa cells.

### Validation of the Results of Hybrid Capture by Southern Blot Hybridization

To confirm the specificity of the coupled amplification/hybrid capture detection assay, the amplification reaction products were analyzed by electrophoresis in agarose gels followed by Southern blot hybridization. Ethidium bromide stained agarose gels of the products obtained when EBV 1 and EBV 6 were used as primers to amplify a portion of the IR1 sequence in pCF1 showed a fragment of approximately 250 bp. This fragment was not observed in the amplification products from pAK8 DNA or Ramos cell lysates. The electrophoretically resolved DNAs were transferred to a Biodyne B nylon membrane and hybridized with the EBV 5 oligonucleotide labeled by 3'- terminal addition of biotin-11-dUTP. The biotin moieties were detected by a colorimetric assay using a complex of streptavidin and biotinylated alkaline phosphatase in the presence of nitroblue tetrazolium and 5-bromo-4-chloro-3-indolyl phosphate. The 250 bp fragment was the major band of hybridization, thus validating the results obtained in the hybrid capture assay.

### Example 2

To examine the utility of these methodologies for the determination of the presence of Human Immunodeficiency Virus (HV I) (also, HTLV III) nucleic acid sequences in samples, human peripheral blood cells were seeded (or were not seeded) with decreasing amounts of purified HIV 1 DNA. The samples, without further manipulation or purification, were then subjected to 25 rounds of amplification by polymerase chain reaction using the primers shown in Table 4A. Following amplification reactions, the samples were then examined essentially as described hereinabove regarding hybrid capture using the probes shown in Table 4A. The results shown in Table 4B were obtained.

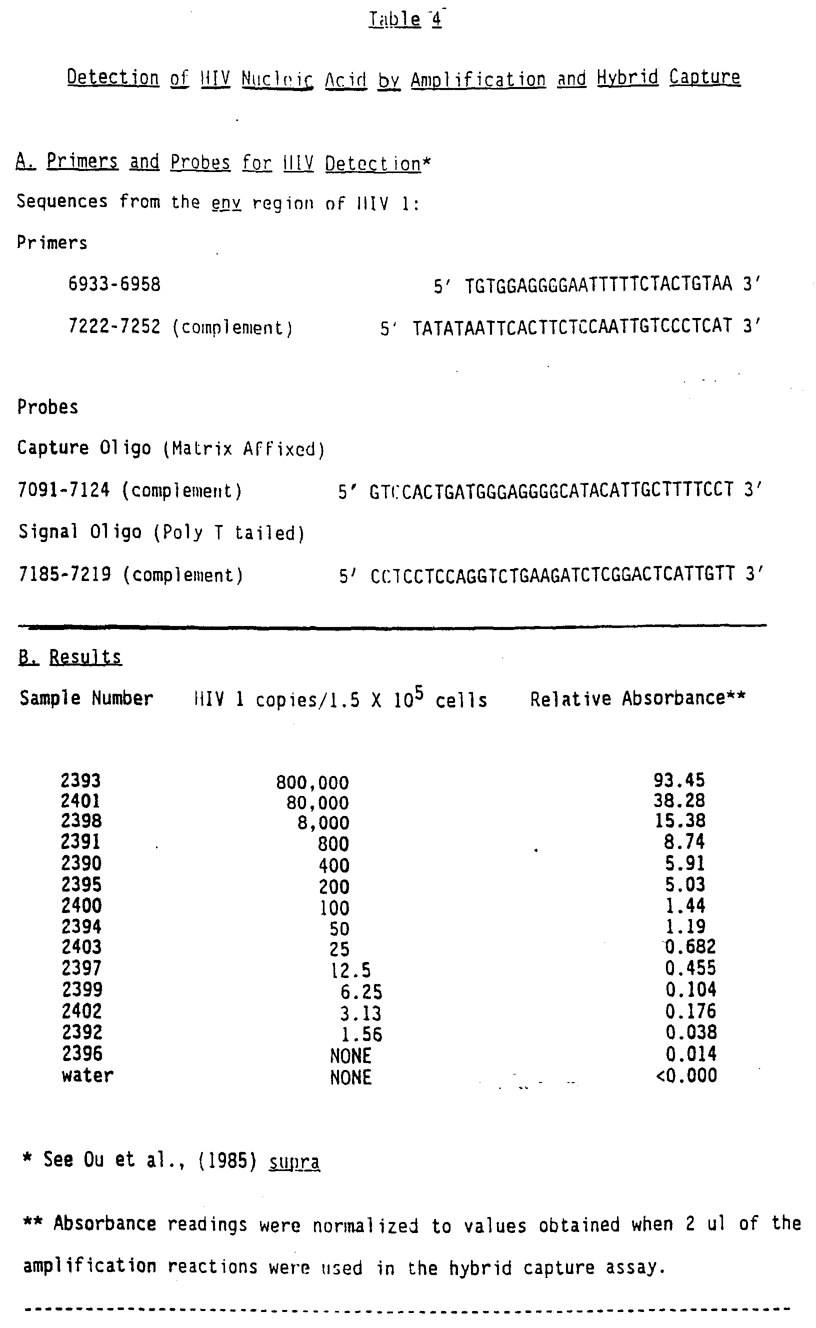

As shown in Table 4B, the limit of detection of HIV nucleic acid was approximately 2 copies in approximately 1.5 x 10⁵ human peripheral blood cells. When portions of the identical samples were subjected to detection by hybridization with a ³²P labeled oligonucleotide probe, electrophoresis and autoradiography, the limit of detection was essentially identical.

These data demonstrate the specificity and sensitivity of the combined amplification and hybrid capture assay. Primers and probes of slightly varying length and/or with minor sequence variations are expected to be interchangeable with the specific sequences used herein.

### Example 3

In order to increase the efficiency of the detection hybridization reactions and to decrease the time required for completion of hybridization, in solution hybridization of amplified target nucleic acid sequence is carried out.

### Labeling of Oligonucleotide Probes

Signal oligonucleotides are 3'-terminally labeled as previously described by Cook et al. (1988), supra. Alternatively they are labeled by incubation in solutions containing 100 mM KPO₄ buffer pH 7.2, 10 mM MgCl₂, 1 mM 2-mercaptoethanol, terminal deoxynucleotide transferase, dATP and biotin-11-dUTP in quantities such that dATP and biotin-11-dUTP are initially present at a molar ratio of 5:1 to 9:1 and the sum of the dATP concentration plus the biotin-11-dUTP concentration is 200 times the concentration of oligonucleotide. Reactions are allowed to proceed for 2-20 hours at 37°C or until all monomers are incorporated.

Capture oligonucleotides are labeled by 3' terminal addition of poly dT as described above. Ligation techniques may also be used, but such methodologies are more complex. Alternatively, capture oligonucleotides can be labeled by incorporation of dinitrophenyl residues in the form of DNP-dUTP. Alternatively, a capture oligonucleotide can be labeled chemically following automated synthesis in the presence of allylamine dUMP phosphoramidites (see Cook et al. (1988), supra). Following synthesis and work up, the allylamine residues are then labeled by reaction with 2,4-dinitrofluorobenzene.

### Capture Matrices and Detection

Poly dA capture wells are prepared by rinsing the wells of Immulon 2 microtiter plates twice with 1M ammonium acetate. Two hundred ng of poly dA (Pharmacia Fine Chemicals, Piscataway; NJ) are added to the wells in 1M ammonium acetate in volumes of 50 ul. Plates are incubated at 37°C in a dry incubator until the wells are dry (12 or more hours). Plates are sealed and are stored at 2-8°C until used. Immediately before use, plates containing affixed poly dA are rinsed twice with 2X SSC and are then allowed to stand for 5 to 60 minutes in 2X SSC containing 0.1% Triton X-100.

Capture wells containing antibodies, such as rabbit anti-DNP IgG, are prepared by incubating an appropriate amount of antibody, diluted in 50 ul of 50 mM bicarbonate buffer, in the wells of Immulon 2 plates for 2 to 4 hours at 37°C. Plates are stored covered at 2-8°C until used. Immediately before use, plates containing affixed antibody are rinsed twice with 0.5X SSC containing 0.1% Triton X-100.

Samples containing amplified copy numbers of target nucleic acid to be analyzed are heat denatured at 92°C for 10 minutes, quick chilled and diluted in sterile water. The diluted samples are added to hybridization solutions, such as 20% formamide, 1X SSPE, 2% dextran sulfate, 1% Triton X-100 and 50 ng/ml of signal oligodeoxynucleotide and an appropriate amount of capture oligonucleotide. For example, a dATP-, biotin-11-dUTP-tailed signal probe is used in conjunction with a poly dT-tailed capture probe, or in conjunction with a DNP-labeled capture probe. Capture probe amounts are previously titrated so that all capture probe, regardless of whether it becomes hybridized to target sequences, will be bound by the capturing agent in a single capturing well of the microtiter plate. Hybridization reactions (100 ul) are allowed to proceed for 5 to 30 minutes at room temperatures and then samples are diluted with 400 ul of 0.05X - 0.1X SSC containing 0.1% Triton X-100. Aliquots of the diluted hybridization reaction are added to wells of the microtiter plate. When poly dA capture wells are used and a poly T-tailed capture probe is used, samples are incubated in the wells for 2 to 5 minutes prior to washing with 0.05X-0.1X SSC containing 0.1% Triton X-100. When anti-DNP antibody is used with DNP-labeled capture probes, wells are incubated 10-60 minutes prior to washing with 0.05X - 0.1X SSC containing 0.1% Triton X-100. Detek 1-hrp can be used to determine the amount of hybrid as described above.

Matrices such as antibody- or polynucleotide-modified latex or polystyrene beads can be used as capture matrices in similar amplification/sandwich capture assays.

### Example 4

Epidemiological studies on the spread of AIDS and the spread of particular strains of HIV are extremely important for establishing the range and distribution of the virus throughout the world's population. In order to monitor the spread of the disease as well as to monitor the progress of the disease in a particular patient, and, perhaps more importantly, in order to protect the world's blood supply it is critical to determine the presence or absence of HIV nucleic acid sequences. While amplification methodologies such as PCR have radically improved abilities to perform such studies, it has been difficult to determine the accuracy of negative results of these assays.

An estimate of the validity of negative results of nucleic acid amplification and detection can be achieved only by accurately determining the amount of HIV DNA in the range of individuals exposed to HIV. Recent studies have suggested that CD4(+) T cells act as the reservoir for latent HIV proviral DNA and that the number of these cells harboring proviral DNA is estimated to be 1/40,000 to 1/100 in asymptomatic seropositive individuals and 1/100 in AIDS patients. See, Psallidopoulos, et al., Integrated Proviral Human Immunodeficiency Virus Type I is Present in CD4+ Peripheral Blood Lymphocytes in Healthy Seropositive Individuals, J. Virology, 63:4626-4631 (1989) and Schnittman, et al., The Reservoir for HIV-1 in Human Peripheral Blood is a T cell that Maintains Expression of CD4, Science, 245:305-308 (1989).

In these studies, ACH2 cells, which contain 1 integrated copy of HIV-1 per cell, were used as a standard for the amplification/detection procedure. As such, the standard fails to account for variation in amplification efficiencies from reaction to reaction. To accurately establish the range of HIV genomes per cell in patient specimens, the efficiency of the amplification in each reaction must be determined. Simultaneous amplification of a known standard sequence such as Beta-globin or glucose-6-phosphate dehydrogenase in the same reaction mixture as the sequence of interest, provides such information.

In Example 2 the limit of detection of amplified HIV nucleic acids was found to be 2 copies of HIV DNA in 150,000 PBLs regardless of whether detection was by the methods disclosed herein or by standard ³²P methodologies. This limit of sensitivity required an efficiency of amplification of at least 70 to 30%. In performing similar tests on clinical samples, readings that are in the range of background to 1-3 copies/150,000 PBLs would have to be interpreted as inconclusive. Low and/or negative readings are the result of either the absence of HIV DNA or the inefficient amplification of the HIV DNA that is present. If the efficiency of each amplification reaction was known, low and/or negative readings could be conclusively interpreted. For example, if the efficiency of an amplification reaction on a clinical sample was known to be 85% and the HIV detection results indicated « 2 copies of HIV DNA (i.e., were insufficiently above the background controls), the absence of HIV DNA (in 150,000 PBLs) could be concluded. If, on the other hand, the efficiency of the amplification reaction was known to be 50% and the HIV sequence detection indicated 0 to 3 copies of HIV, the result would be known to be inconclusive and the procedure could be repeated to achieve the requisite amplification efficiency.

While it is obvious that the inclusion of a known standard in amplification reactions can be used to estimate the efficiency of each amplification reaction, heretofore, no simple means for accomplishing this has been described. The methods disclosed herein are readily adaptable to such procedures. In Example 2, when patient specimens are subjected to amplification by the polymerase chain reaction methods using disrupted, unpurified peripheral blood lymphocyte (PBL) cells as sample material, two sets of primers, one set for HIV sequences and a second set for a marker sequence, can be included in the PCR reactions. The second set of primers is chosen from among gene sequences that are known to be present in the normal genomes of PBLs in one copy per cell. An example of such a gene sequence is the Beta-globin gene. Another example is a gene for enzymes of intermediary metabolism, such as the glucose-6-phosphate dehydrogenase gene. If the gene for Beta-globin is used, sequences of primers as described in Saiki et al., Enzymatic amplification of B-globin genomic sequences and restriction site analysis for diagnosis of sickle cell anemia, Science, 230:1350-1354 (1985) would be appropriate. A pair of nonoverlapping, single-stranded pulynucleotide probes are chosen from sequences located between these primers. They are chosen to be complementary to one strand or the other and are used to detect the amplified copy number of the Beta-globin sequences. Detection is carried out by procedures used in detection of target nucleic acids.

An amplification reaction for detection of HIV 1 sequences and for Beta-globin sequences is carried out on suspensions of PBLs in the presence of the primers noted in Table 4 for HIV 1 and in the presence of the primers for Beta-globin sequences as noted in Saiki et al., (1985) supra. Following amplification, hybridization to probes is performed essentially as described above. Two poly T-tailed probes are added to hybridization solutions. The T-tailed probe for HIV in Table 4B is used as is a T-tailed probe for Beta-globin, chosen as described above. Portions of the hybridization reaction are added to individual wells of a microtiter plate. One set of wells contains the probe for HIV 1 as noted in Table 4B and another set of wells has a probe, chosen as described above, for Beta-globin. Specific dilutions of the amplified nucleic acids are added to the hybridization mixture and then dilutions are added to the wells of the microtiter plate. The amplified nucleic acid sequences are determined. From the absorbance readings obtained when the results are developed, the efficiency of the amplification reaction is established and the validity of negative results for the target sequence of interest is determined.

These procedures can also be used to detect the presence of a second target nucleic acid sequence of interest. For example, the primers as described above for EBV and for HIV can be used in a single amplification reaction and the wells of a microtiter plate can contain either the EBV-specific or the HIV-specific oligonucleotide and the presence of either HIV or EBV or both can be established in a single amplification reaction and in a single detection operation.

### Example 5

In Example 4, studies determining the amount of HIV DNA in infected individuals may confirm the range of 1 genome copy in 10,000 to 1,000,000 PBLs, depending on the stage of the infection. If it was possible to simply improve the efficiency of the amplification reactions, it would be possible to consistently use 10⁶ or more cells in each amplification reaction to perform such a study. Alternatively, it is possible to increase the number of rounds of amplification from 25 to 35 (or more) to perform such a study. However, the efficiency of amplification tends to decrease as the amount of crude material input into the amplification reaction is increased. In addition, increasing the number of rounds of amplification beyond 25-30 often leads to the production of non-target-specific sequence production.

A way to circumvent these difficulties is to purify the nucleic acid from the sample. Unfortunately, purification of nucleic acids from many samples is difficult, tedious, and may lead to cross contamination of the samples. Furthermore, purification of the nucleic acid from a sample to be tested often requires the use of hazardous materials, e.g. phenol. In other cases, purification of the nucleic acid from a sample may be required because the conditions and materials that are required for sample disruption and release of the nucleic acid are incompatible with enzymatic amplification of nucleic acids. For example, phenol, detergents and reducing agents are used to disrupt samples for testing for the presence of Mycobacterium tuberculosis or M. avium nucleic acids in sputum samples. In such cases, the nucleic acid must be freed of these disruption agents prior to initiating the amplification reactions.

A simple method for circumventing these difficulties is the pre-capturing or pre-selecting of the sequences of interest. Such procedures offer the opportunity to use almost any amount of sample and the opportunity to use conditions and materials for disrupting the sample that are ordinarily incompatible with enzymatic amplification reactions.

By pre-capturing the prospective target nucleic acid from a disrupted sample, the efficiency of amplification can be made more consistent and the materials used to disrupt some samples can be readily removed prior to initiating the enzymatic amplification reactions. One need not be concerned that the target nucleic acid is at such a low concentration that the hybridization reaction would require inordinately long times. In performing amplification reactions, the first round of amplification requires the hybridization (annealing) of the primers with the target nucleic acid. This usually requires less than 5 minutes in the currently used protocols. Performing the initial annealing reaction between the nucleic acid in a disrupted sample and matrix-bound or capturable pre-capture probes allows one to free the target nucleic acid from the remainder of the sample and from the sample disruption agents.

As in Example 1, six to eight different, nonoverlapping probe/primer oligonucleotides are chosen and synthesized for a particular target nucleic acid sequence. These are shown as oligonucleotides A-H in Fig. 5A. To form the pre-capturing probes when the target is DNA, two of these oligonucleotides (oligonucleotides A and H of Fig. 5A) (one oligonucleotide if the target is originally single-stranded, i.e., RNA), are then linked through the 5' termini to Sephacryl or to controlled pore glass or other support as described by Ghosh and Musso (1987), supra. In one procedure, disrupted sample is allowed to hybridize with bead-linked oligonucleotide pre-capturing probes. Following this hybridization, the beads are collected by sedimentation or by filtration and are washed with a simple saline buffer to remove the remainder of the sample and the components of the disrupting solutions. Once the beads are freed of these materials, the beads are suspended in amplification reaction mixtures (buffer, dNTPs and polymerases). Amplification is then carried out as usual. In Fig. 5B, three rounds of amplification are shown. Each round of amplification in this system generates target sequences of interest that are linked to the beads. Following amplification, labeled signal oligonucleotides B,C or D and/or E, F or G can be used to detect the bead-linked, amplified nucleic acid target sequences.

In another method, the pre-capturing oligonucleotides are linked to a matrix as above , but the 3' ends of the oligonucleotides A and H are modified so that they cannot serve as primers for polymerases (shown in Fig. 5C as a darkened ball on oligonucleotides A and H); e.g., a dideoxynucleotide is placed at the 3' termini, or the 3' OH is modified by methylation or glycosylation. In this case, hybridization with the nucleic acid is also followed by washing of the hybrids bound to the beads. Once the beads and hybrids are washed, primers (shown in Fig. 5C as oligonucleotides B and G) are added with the other components of the amplification reaction (buffer, dNTPs and polymerase). Amplification is then carried out as usual. Fig. 5C shows three rounds of amplification in which all amplified nucleic acid is free in solution and not linked to the beads although the beads, and their bound oligonucleotides, are present during the amplification reaction. Following amplification, probe pairs C and D and/ or E and F are used for detection as described above.

Pre-capture of target sequences can also be accomplished by in solution hybridization procedures. Such pre-capture probes are modified by a first pre-capture moiety. Following annealing with target nucleic acids, the hybrids (as well as unhybridized pre-capture probes) are contacted with a matrix-bound second pre-capturing moiety which specifically interacts with the first pre-capturing moiety. For example, pre-capture oligonucleotides A and H of Fig. 5 could be labeled at their 3' termini with poly T as described above, or at their 5' termini during synthesis. The 3' termini of the "tailed" pre-capture probes are then blocked as described above. Following annealing to the nucleic acids in the disrupted sample, the hybrids (and all pre-capture probes A and H) are contacted with poly A or poly dA linked to Sephacryl, latex, polystyrene, CPG or other solid matrix. The hybrids are washed as above to free them of the remainder of the sample and the sample disruption agents. The matrix containing the hybrids is then suspended in amplification reaction mixtures containing primers B and G as shown in Fig. 5C. Amplification is carried out as usual and the amplified nucleic acid is detected as for the amplified material in Fig. 5C.

## Claims

1. A composition for the determination of a single-stranded target nucleic acid in a sample to be tested which comprises:
(i) an oligonucleotide primer for each single-stranded target nucleic acid of interest, which primer is capable of annealing selectively to a complementary portion of its target nucleic acid;
(ii) mononucleotides of each of the four nucleic acid bases;
(iii) nucleic acid polymerases capable of producing an extension product from the primer and mononucleotides which is complementary to the single strand of target nucleic acid to which the primer is annealed;
(iv) a first polynucleotide probe comprising at least one component of a labeling system attached to a first single-stranded polynucleotide segment which is hybridizable with a first portion of the target nucleic acid;
(v) a second polynucleotide probe comprising a first entity attached to a second single-stranded polynucleotide segment which is hybridizable with a second portion of the target nucleic acid; and
(vi) a matrix having attached thereto a second entity which is specifically bindable with the first entity.

2. The composition of claim 1 wherein each of the oligonucleotide primers is at least a decanucleotide.

3. The composition of claim 1 wherein the nucleic acid polymerase is a DNA polymerase, a reverse transcriptase, or a reverse transcriptase and a DNA polymerase.

4. The composition of claim ¹ wherein the labeling system component is one partner of a specific binding pair and wherein the composition further comprises a binding partner therefor bound to a detectable moiety.

5. The composition of claim ¹ wherein one entity is a ligand and the other entity is a receptor therefor.

6. The composition of claim ¹ wherein the first and second entities are complementary polynucleotides.

7. The composition of claim ¹ wherein one entity is an antigen or hapten and the other entity is an antibody or specific binding fragment thereof for the antigen or hapten, or one entity is a hormone and the other entity is a receptor therefor, or one entity is an enzyme and the other entity is an inhibitor thereof, or one entity is an apoenzyme and the other entity is a cofactor therefor, or one entity is a sugar and the other entity is a lectin which is specifically bindable therewith, or one entity is avidin or streptavidin and the other entity is biotin or a binding analog thereof.

8. A method for the determination of single-stranded target nucleic acid in a sample to be tested, which method comprises:
(a) amplifying the copy number of the target nucleic acid;
(b) contacting, under conditions permissive of hybridization, the amplified copy number of the target nucleic acid with;
(i) a first polynucleotide probe comprising at least one component of a labeling system attached to a first single-stranded polynucleotide segment which is hybridizable with a first portion of the target nucleic acid;
(ii) a second polynucleotide probe comprising a first entity attached to a second single-stranded polynucleotide segment which is hybridizable with a second portion of the target nucleic acid; and
(iii) a matrix having attached thereto a second entity which is specifically bindable with the first entity;
(c) permitting hybridization of the first and second probes with any of the amplified copy number of the target nucleic acid present and binding of the first entity with the second entity to produce a bound complex;
(d) separating, if necessary, the bound complex from any unbound nucleic acid; and
(e) determining the presence or absence of the amplified target nucleic acid.

9. The method of claim 8 wherein amplifying the copy number of the target nucleic acid is by polymerase chain reaction or by using a Q Beta replicase.

10. The method of claim 8 wherein the amplified copy number of target nucleic acid is simultaneously contacted with the first and second probes so as to form the complex and, thereafter, the second entity is contacted with the complex.

11. The method of claim 8 wherein the first probe and the amplified copy number of target nucleic acid are contacted with a matrix to which the second probe has been bound by the first and second entities.

12. The method of claim 8 wherein the first and second entities are complementary polynucleotides, or one entity is a ligand and the other entity is a receptor therefor, or one entity is an antigen or hapten and the other entity is an antibody or specific binding fragment thereof for the antigen or hapten, or one entity is a hormone and the other entity is a receptor therefor, or one entity is an enzyme and the other entity is an inhibitor thereof, or one entity is an apoenzyme and the other entity is a cofactor therefor, or one entity is a sugar and the other entity is a lectin which is specifically bindable therewith, or one entity is avidin or streptavidin and the other entity is biotin or a binding analog thereof.

13. A composition for determining single-stranded HIV nucleic acid in a sample to be tested, which composition comprises:
(a) oligonucleotide primers having the following sequences 5′ TGTGGAGGGGAATTTTTCTACTGTAA 3′ and 5'. TATATAATTCACTTCTCCAATTGTCCCTCAT 3'
(b) mononucleotides of each of the four nucleic acid bases;
(c) nucleic acid polymerases capable of producing an extension product from the primers and mononucleotides which is complementary to the single strand of HIV nucleic acid to which the primer is annealed;
(d) a first polynucleotide probe comprising a universal label-capturing moiety attached to a first single-stranded polynucleotide segment which has a sequence selected from the group consisting of 5' CCTCCTCCAGGTCTGAAGATCTCGGACTCATTGTT 3' and 5' GTCCACTGATGGGAGGGGCATACATTGCTTTTCCT 3'
(e) a second polynucleotide probe having the other of the sequences;
(f) a matrix to which the second polynucleotide probe is affixed; and
(g) a universal label which is capturable by the first universal label-capturing moiety contained in the first polynucleotide probe when the first polynucleotide probe is captured by hybridization to the amplified target nucleic acid sequences which is in turn captured by hybridization with the matrix-affixed the second polynucleotide probe.

14. The composition of claim ¹³ wherein the universal label-capturing moiety is a poly-T sequence.

15. The composition of claim ¹³ wherein the universal label-capturing moiety is avidin or streptavidin and its binding partner is biotin or a binding analog thereof.

16. A method for determining single-stranded HIV nucleic acid in a sample to be tested, which method comprises:
(a) amplifying the copy number of the HIV nucleic acid using primers having the following sequences 5' TGTGGAGGGGAATTTTTCTACTGTAA 3' and 5' TATATAATTCACTTCTCCAATTGTCCCTCAT 3'
(b) contacting so as to permit hybrid formation of the amplified copy number of HIV nucleic acid with a first polynucleotide probe comprising a first universal label-capturing moiety attached to a first single-stranded polynucleotide segment which has a sequence selected from the group consisting of 5' CCTCCTCCAGGTCTGAAGATCTCGGACTCATTGTT 3' and 5′ GTCCACTGATGGGAGGGGCATACATTGCTTTTCCT 3′
(c) contacting the hybrid so-formed, under conditions permissive of hybridization, with a matrix-affixed second polynucleotide probe comprising a second single-stranded polynucleotide segment having the other of the sequences; and
(d) permitting hybridization of the hybrid with the matrix-affixed second probe;
(e) separating, if necessary, bound hybrid from any unbound nucleic acid; and
(f) determining the presence or absence of the amplified HIV nucleic acid.

17. A composition for determining single-stranded HIV nucleic acid in a sample to be tested, which composition comprises:
(a) oligonucleotide primers having the sequences 5′ TGTGGAGGGGAATTTTTCTACTGTAA 3′ and 5' TATATAATTCACTTCTCCAATTGTCCCTCAT 3'
(b) mononucleotides of each of the four nucleic acid bases;
(c) nucleic acid polymerases capable of producing an extension product from the primer and mononucleotides which is complementary to the single strand of HIV nucleic acid to which the primer is annealed;
(d) a first polynucleotide probe comprising at least one component of a labeling system attached to a first single-stranded polynucleotide segment having a sequence selected from the group consisting of 5' CCTCCTCCAGGTCTGAAGATCTCGGACTCATTGTT 3' and 5' GTCCACTGATGGGAGGGGCATACATTGCTTTTCCT 3'
(e) a second polynucleotide probe comprising a first entity attached to a second single-stranded polynucleotide segment having the other of the sequences; and
(f) a matrix having attached thereto a second entity which is specifically bindable with the first entity.

18. A method for determining single-stranded HIV nucleic acid in a sample to be tested, which method comprises:
(a) amplifying the copy number of the target nucleic acid using primers having the following sequences 5′ TGTGGAGGGGAATTTTTCTACTGTAA 3′ and 5′ TATATAATTCACTTCTCCAATTGTCCCTCAT 3′
(b) contacting so as to permit hybrid formation of the amplified copy number of the HIV nucleic acid with:
(i) a first polynucleotide probe comprising at least one component of a labeling system attached to a first single-stranded polynucleotide segment which has a sequence selected from the group consisting of 5′ CCTCCTCCAGGTCTGAAGATCTCGGACTCATTGTT 3′ and 5′ GTCCACTGATGGGAGGGGCATACATTGCTTTTCCT 3′
(ii) a second polynucleotide probe comprising a first entity attached to a second single-stranded polynucleotide segment having the other of the sequences; and
(iii) a matrix having attached thereto a second entity which is specifically bindable with the first entity;
(c) permitting hybridization of the first and second probes with any of the amplified copy number of HIV nucleic acid present and binding of the first entity with the second entity to produce a bound complex;
(d) separating, if necessary, the bound complex from any unbound nucleic acid; and
(e) determining the presence or absence of amplified HIV nucleic acid.

19. A composition for determining single-stranded Epstein-Barr virus nucleic acid in a sample to be tested, which composition comprises:
(a) oligonucleotide primers having the following sequences 5′ TTCGCGTTGCTAGGCCACCTTCTCAGT 3′ and 5' CAAGACGAGGGAGGGAAGGGGAAAAGT 3'
(b) mononucleotides of each of the four nucleic acid bases;
(c) nucleic acid polymerases capable of producing an extension product from the primers and mononucleotides which is complementary to the single strand of Epstein-Barr virus nucleic acid to which the primer is annealed;
(d) a first polynucleotide probe comprising a universal label-capturing moiety attached to a first single-stranded polynucleotide segment which has a sequence selected from the group consisting of 5' GATGCGACCAGAAATAGCTGCAGGACC 3' and 5' CCTCTTACATTTGTGTGGACTCCTGGC 3'
(e) a second polynucleotide probe having the other of the sequences;
(f) a matrix to which the second polynucleotide probe is affixed; and
(g) a universal label capturable by the first universal label-capturing moiety when the first polynucleotide probe is captured by hybridization to an amplified Epstein-Barr nucleic acid sequence which is captured by hybridization with the matrix-affixed second polynucleotide probe.

20. The composition of claim ^{**19**} wherein the universal label-capturing moiety is a poly-T sequence.

21. The composition of claim ^{**19**} wherein the universal label-capturing moiety is avidin or streptavidin and its binding partner is biotin or a binding analog thereof.

22. A method for the determining single-stranded Epstein-Barr virus nucleic acid in a sample to be tested, which method comprises:
(a) amplifying the copy number of the Epstein-Barr virus nucleic acid using primers having the following sequences 5′ TTCGCGTTGCTAGGCCACCTTCTCAGT 3′ and 5' CAAGACGAGGGAGGGAAGGGGAAAAGT 3'
(b) contacting so as to permit hybrid formation of the amplified copy number of Epstein-Barr virus nucleic acid with a first polynucleotide probe comprising a first universal label-capturing moiety attached to a first single-stranded polynucleotide segment which has a sequence selected from the group consisting of 5′ GATGCGACCAGAAATAGCTGCAGGACC 3′ and 5′ CCTCTTACATTTGTGTGGACTCCTGGC 3′
(c) contacting the hybrid, under conditions permissive of hybridization, with a matrix-affixed second polynucleotide probe comprising a second single-stranded polynucleotide segment having the other of the sequences; and
(d) permitting hybridization of the hybrid with the matrix-affixed second probe;
(e) separating, if necessary, bound hybrid from any unbound nucleic acid; and
(f) determining the presence or absence of Epstein-Barr virus nucleic acid.

23. A composition for determining single-stranded Epstein-Barr virus nucleic acid in a sample to be tested, which composition comprises:
(a) oligonucleotide primers having the sequences 5′ TTCGCGTTGCTAGGCCACCTTCTCAGT 3′ and 5' CAAGACGAGGGAGGGAAGGGGAAAAGT 3'
(b) mononucleotides of each of the four nucleic acid bases;
(c) nucleic acid polymerases capable of producing an extension product from the primer and mononucleotides which is complementary to the single strand of Epstein-Barr virus nucleic acid to which the primer is annealed;
(d) a first polynucleotide probe comprising at least one component of a labeling system attached to a first single-stranded polynucleotide segment having a sequence selected from the group consisting of 5′ GATGCGACCAGAAATAGCTGCAGGACC 3′ and 5' CCTCTTACATTTGTGTGGACTCCTGGC 3'
(e) a second polynucleotide probe comprising a first entity attached to a second single-stranded polynucleotide segment having the other of the sequences; and
(f) a matrix having attached thereto a second entity which is specifically bindable with the first entity.

24. A method for determining single-stranded Epstein-Barr virus nucleic acid in a sample to be tested, which method comprises:
(a) amplifying the copy number of the Epstein-Barr nucleic acid using primers having the following sequences 5′ TTCGCGTTGCTAGGCCACCTTCTCAGT 3′ and 5' CAAGACGAGGGAGGGAAGGGGAAAAGT 3'
(b) contacting so as to permit hybrid formation of the amplified copy number of target nucleic acid with:
(i) a first polynucleotide probe comprising at least one component of a labeling system attached to a first single-stranded polynucleotide segment which has a sequence selected from the group consisting of 5' GATGCGACCAGAAATAGCTGCAGGACC 3' and 5' CCTCTTACATTTGTGTGGACTCCTGGC 3'
(ii) a second polynucleotide probe comprising a first entity attached to a second single-stranded polynucleotide segment having the other of the sequences; and
(iii) a matrix having attached thereto a second entity which is specifically bindable with the first entity;
(c) permitting hybridization of the first and second probes with any of the amplified copy number of Epstein-Barr virus nucleic acid present and binding of the first entity with the second entity to produce a bound complex;
(d) separating, if necessary, the bound complex from any unbound nucleic acid; and
(e) determining the presence or absence of the amplified Epstein-Barr virus nucleic acid.

25. A composition for determining single-stranded target nucleic acid in a sample, which composition comprises:
(a) a matrix-attached capture polynucleotide for each single-stranded target nucleic acid of interest, which polynucleotide is capable of annealing selectively to a complementary portion of its target nucleic acid as well as serving as a primer for its target-nucleic acid;
(b) mononucleotides of each of the four nucleic acid bases;
(c) nucleic acid polymerases capable of producing an extension product from the capture polynucleotide/primer and mononucleotides which is complementary to the single strand of target nucleic acid to which the capture polynucleotide/primer is annealed to provide matrix-bound amplified nucleic acid sequences;
(d) a polynucleotide probe comprising at least one component of a labeling system attached to a single-stranded polynucleotide segment which is hybridizable with a portion of the amplified, matrix-bound target nucleic acid.

26. A method for determining single-stranded target nucleic acid in a sample to be tested, which method comprises:
(a) contacting so as to permit hybrid formation between the single-stranded target nucleic acid and a matrix-affixed capture polynucleotide for each single-stranded target nucleic acid of interest;
(b) separating the hybridized target nucleic acid from the sample;
(c) amplifying the copy number of the target nucleic acid to generate matrix bound amplified copy numbers of target nucleic acid;
(d) contacting, under conditions permissive of hybridization, the amplified copy numbers with a polynucleotide probe comprising at least one component of a labeling system attached to a single-stranded polynucleotide segment which is hybridizable with a portion of the amplified target nucleic acid;
(e) permitting hybridization to form a complex between amplified target nucleic acid and probe;
(f) separating, if necessary, the bound probe from any unbound nucleic acid; and
(g) determining the presence or absence of the bound amplified target by observing the bound label.

27. A composition for determining single-stranded target nucleic acid in a sample to be tested, which composition comprises:
(a) a matrix-attached capture polynucleotide for each single-stranded target nucleic acid of interest, which polynucleotide is capable of annealing selectively to a complementary portion of its target nucleic acid;
(b) an oligonucleotide primer for each single-stranded target nucleic acid of interest, which primer is capable of annealing selectively to a complementary portion of its target nucleic acid;
(c) mononucleotides of each of the four nucleic acid bases;
(d) nucleic acid polymerases capable of producing an extension product from the primer and mononucleotides which is complementary to the single strand of target nucleic acid to which the primer is annealed;
(e) a first polynucleotide probe comprising at least one component of a labeling system attached to a first single-stranded polynucleotide segment which is hybridizable with a first portion of the target nucleic acid;
(f) a second polynucleotide probe comprising a first entity attached to a second single-stranded polynucleotide segment which is hybridizable with a second portion of the target nucleic acid; and
(g) a matrix having attached thereto a second entity which is specifically bindable with the first entity.

28. A method for determining single-stranded target nucleic acid in a sample to be tested, which method comprises:
(a) contacting the single-stranded target nucleic acid, under conditions permissive of hybridization, with at least one matrix-attached capture polynucleotide for each single-stranded target nucleic acid of interest, which polynucleotide is capable of annealing selectively to a complementary portion of its target nucleic acid;
(b) separating the hybridized target nucleic acid from the sample;
(c) amplifying the copy number of the target nucleic acid by adding primer non-overlapping with capture sequences to generate unbound amplified copy numbers of target nucleic acid;
(d) contacting, so as to form a complex, the unbound amplified target nucleic acid with:
(i) a first polynucleotide probe comprising at least one component of a labeling system attached to a first single-stranded polynucleotide segment which is hybridizable with a first portion of the target nucleic acid;
(ii) a second polynucleotide probe comprising a first entity attached to a second single-stranded polynucleotide segment hybridizable with a second portion of the target nucleic acid;
(e) contacting the complex with a matrix having attached thereto a second entity which is specifically bindable with the first entity;
(f) separating, if necessary, the bound probe from any unbound nucleic acid; and
(g) determining the presence or absence of the bound amplified target nucleic acid by observing the presence of bound label.

29. A composition for determining single-stranded HIV nucleic acid in a sample to be tested, which composition comprises:
(a) matrix-attached capture polynucleotides having the following sequences 5′ TGTGGAGGGGAATTTTTCTACTGTAA 3′ and 5' TATATAATTCACTTCTCCAATTGTCCCTCAT 3'
(b) mononucleotides of each of the four nucleic acid bases;
(c) nucleic acid polymerases capable of producing an extension product from the primers and mononucleotides which is complementary to the single strand of HIV nucleic acid to which the primer is annealed;
(d) a first polynucleotide probe comprising at least one component of a labeling system attached to a single-stranded polynucleotide segment which has a sequence selected from the group consisting of 5′ CCTCCTCCAGGTCTGAAGATCTCGGACTCATTGTT 3′ and 5′ GTCCACTGATGGGAGGGGCATACATTGCTTTTCCT 3′;
(e) a second polynucleotide probe having the other of the sequences.

30. A method for determining single-stranded HIV nucleic acid in a sample to be tested, which method comprises:
(a) contacting the single-stranded HIV nucleic acid so as to form a hybrid with matrix-affixed capture polynucleotides/primers having the following sequences 5'TGTGGAGGGGAATTTTTCTACTGTAA 3' and 5′ TATATAATTCTCTTCTCCAATTGTCCCTCAT 3′
(b) separating the so-hybridized HIV nucleic acid from the sample;
(c) amplifying the copy number of the HIV nucleic acid to generate matrix-bound amplified copy numbers of HIV nucleic acid;
(d) contacting, under conditions permissive of hybridization, the amplified copy number of HIV nucleic acid with a polynucleotide probe comprising at least one component of a labeling system attached to a single-stranded polynucleotide segment selected from the group consisting of 5′ CCTCCTCCAGGTCTGAAGATCTCGGACTCATTGTT 3′ and 5′ GTCCACTGATGGGAGGGGCATACATTGCTTTTCCT 3′;
(f) permitting hybridization of the probe with the matrix-affixed HIV nucleic acid;
(g) separating, if necessary, bound hybrid from any unbound nucleic acid; and
(h) determining the presence or absence of the amplified HIV nucleic acid by observing the presence or absence of bound label.

31. A composition for determining single-stranded target nucleic acid and for confirming the efficiency of amplification thereof in a sample containing a marker nucleic acid and suspected of containing target nucleic acid, which composition comprises:
(a) an oligonucleotide primer for each single-stranded target nucleic acid in a reaction zone, which primer is capable of annealing selectively to a complementary portion of the single-stranded target nucleic acid;
(b) an oligonucleotide primer for each single-stranded marker nucleic acid, whose copy number in the sample is known, in the reaction zone, which primer is capable of annealing selectively to a complementary portion of the single-stranded marker nucleic acid;
(c) mononucleotides of each of the four nucleic acid bases;
(d) nucleic acid polymerases capable of producing in the reaction zone extension products from the primers and mononucleotides which are complementary to the single strand of target and marker nucleic acids, respectively, to which the primers are annealed;
(e) a first polynucleotide target probe comprising at least one component of a first labeling system attached to a first single-stranded polynucleotide segment hybridizable with a first portion of the target nucleic acid;
(f) a second polynucleotide target probe bound to a first test zone separate from the reaction zone and comprising a second single-stranded polynucleotide segment hybridizable with a second portion of the target nucleic acid;
(g) a first polynucleotide marker probe comprising at least one component of a labeling system attached to a first single-stranded polynucleotide segment which is hybridizable with a first portion of the marker nucleic acid; and
(h) a second polynucleotide marker probe bound to a second test zone separate from the reaction zone and the first test zone and comprising a second single-stranded polynucleotide segment hybridizable with a second portion of the marker nucleic acid.

32. A method for determining a single-stranded target nucleic acid and for confirming the efficiency of amplification thereof in a sample containing a marker nucleic acid and suspected of containing target nucleic acid, which method comprises:
(a) contacting the sample in a reaction zone with an oligonucleotide primer capable of annealing selectively to a complementary portion of the single-stranded target nucleic acid;
(b) contacting the sample in the reaction zone containing a known copy number of marker nucleic acid with an oligonucleotide primer capable of annealing selectively to a complementary portion of the single-stranded marker nucleic acid;
(c) amplifying the copy number of the target and marker nucleic acids in the reaction zone by contact with a nucleic acid polymerase;
(d) contacting in the reaction zone, under conditions permissive of hybridization, the amplified copy number of target and marker nucleic acid with:
(i) a first polynucleotide target probe, comprising at least one component of a first labeling system attached to a first single-stranded polynucleotide segment hybridizable with a first portion of the target nucleic acid and
(ii) a first polynucleotide marker probe, comprising at least one component of a second labeling system attached to a first single-stranded polynucleotide segment hybridizable with a first portion of the marker nucleic acid;
(e) permitting hybridization of the first marker and target probes with the amplified copy number of marker nucleic acid and any target nucleic acid which may be present, respectively, so as to form marker and target hybrids;
(f) contacting a first aliquot of the sample so-treated in a first test zone separate from the reaction zone with a second polynucleotide target probe bound to the first test zone so as to permit hybridization of the second target probe with any of the amplified copy number of target nucleic acid present;
(g) contacting a second aliquot of the sample so-treated in a second test zone separate from the reaction zone and the first test zone with a second polynucleotide marker probe bound to the second test zone so as to permit hybridization of the second marker probe with the amplified copy number of marker nucleic acid;
(h) separating, if necessary, from the test zones any unbound nucleic acid; and
(i) determining the copy number of any target nucleic acid present in the first test zone and the copy number of marker nucleic acid in the second test zone.

33. A composition for determining a single-stranded target nucleic acid and for confirming the efficiency of amplification thereof in a sample containing a marker nucleic acid and suspected of containing target nucleic acid, which composition comprises:
(a) an oligonucleotide primer for each single-stranded target nucleic acid in a reaction zone, which primer is capable of annealing selectively to a complementary portion of the single-stranded target nucleic acid;
(b) an oligonucleotide primer for each single-stranded nucleic acid whose copy number in the sample is known, in the reaction zone which primer is capable of annealing selectively to a complementary portion of the single-stranded marker nucleic acid;
(c) mononucleotides of each of the four nucleic acid bases;
(d) nucleic acid polymerases capable of producing in the reaction zone extension products from the primers and mononucleotides which are complementary to the single strands of target and marker nucleic acids, respectively, to which the primers are annealed;
(e) a first polynucleotide target probe comprising at least one component of a first labeling system attached to a first single-stranded polynucleotide segment hybridizable with a first portion of the target nucleic acid;
(f) a second polynucleotide target probe comprising a first entity attached to a second single-stranded polynucleotide segment hybridizable with a second portion of the target nucleic acid;
(g) a target test zone having bound thereto a second entity which is specifically bindable with the first entity;
(h) a first polynucleotide marker probe comprising at least one component of a second labeling system attached to a first single-stranded polynucleotide segment hybridizable with a first portion of the marker nucleic acid;
(i) a second polynucleotide marker probe comprising a third entity attached to a second single-stranded polynucleotide segment hybridizable with a second portion of the marker nucleic acid; and
(j) a target test zone having bound thereto a fourth entity which is specifically bindable with the third entity.

34. The composition of claim 33 wherein the first and second labeling systems are the same and the first and second binding entity pair is different from the third and forth binding entity pair.

35. The composition of claim 33 wherein the first and second labeling systems are distinguishably different and the first and second binding entity pair is the same as the third and forth binding entity pair.

36. A method for determining a single-stranded target nucleic acids and for confirming the efficiency of amplification thereof in a sample containing a marker nucleic acid and suspected of containing target nucleic acid, which method comprises:
(a) contacting the sample with an oligonucleotide primer for the single-stranded target nucleic acid in a reaction zone, which primer is capable of annealing selectively to a complementary portion of the single-stranded target nucleic acid;
(b) contacting the sample with an oligonucleotide primer for the single-stranded marker nucleic acid in the reaction zone,the copy number of which in the sample is known, which primer is capable of annealing selectively to a complementary portion of the single-stranded marker nucleic acid;
(c) amplifying in the reaction zone the copy number of the target and marker nucleic acids in the sample by contacting the sample with a nucleic acid polymerase;
(d) contacting in the reaction zone, under conditions permissive of hybridization, the amplified copy number of target nucleic acid with:
(i) a first polynucleotide target probe comprising a label moiety and a first single-stranded polynucleotide segment hybridizable with a first portion of the target nucleic acid attached to the label moiety;
(ii) a second polynucleotide target probe comprising a second single-stranded polynucleotide segment hybridizable with a second portion of the target nucleic acid and a first entity attached to the second polynucleotide segment;
(e) contacting in the reaction zone, under conditions permissive of hybridization, the amplified copy number of marker nucleic acid with:
(i) a first polynucleotide marker probe comprising a label moiety and a first single-stranded polynucleotide segment hybridizable with a first portion of the marker nucleic acid attached to the label; and
(ii) a second polynucleotide marker probe comprising a second single-stranded polynucleotide segment hybridizable with a second portion of the marker nucleic acid and a third entity attached to the second polynucleotide segment;
(f) permitting hybridization of (i) the first and second target probes with any of the amplified copy number of target nucleic acid present and (ii) the first and second marker probes with the amplified copy number of marker nucleic acid present;
(g) contacting an aliquot of the sample as treated in steps (a)-(f) with a second entity specifically bindable with the first entity in a first test zone which is separate from the reaction zone and to which the second entity is bound;
(h) contacting another aliquot of the sample as treated in steps (a)-(f) with a fourth entity specifically bindable with the third entity in a second test zone which is separate from the reaction zone and first test zone and to which the fourth entity is bound;
(i) separating, if necessary, nucleic acid bound to a test zone from any unbound nucleic acid; and
(j) determining the copy number of the amplified marker nucleic acid and the presence or absence of any target nucleic acid.

37. A composition for determining single-stranded HIV nucleic acid and for confirming the efficiency of amplification thereof in a sample containing a marker nucleic acid, which composition comprises:
(a) HIV oligonucleotide primers useful in a reaction zone having the following sequences 5' TGTGGAGGGGAATTTTTCTACTGTAA 3' and 5′ TATATAATTCACTTCTCCAATTGTCCCTCAT 3′
(b) an oligonucleotide primer for each single-stranded marker nucleic acid whose copy number in the sample is known, which primer is capable of annealing selectively to a complementary portion of its single-stranded marker nucleic acid;
(c) mononucleotides of each of the four nucleic acid bases;
(d) nucleic acid polymerases capable of producing extension products from the primers and mononucleotides which are complementary to the single strand of target and marker nucleic acids, respectively, to which the primers are annealed;
(e) a first polynucleotide HIV probe comprising a label moiety and a first single-stranded polynucleotide segment attached to the label moiety, the first single-stranded polynucleotide segment having a sequence selected from the group consisting of 5′ CCTCCTCCAGGTCTGAAGATCTCGGACTCATTGTT 3′ and 5' GTCCACTGATGGGAGGGGCATACATTGCTTTTCCT 3'
(f) a second polynucleotide HIV probe having the other of the sequences and being bound to a first test zone separate from the reaction zone;
(g) a first polynucleotide marker probe comprising a second label moiety, distinguishable from the target label moiety, and a first single-stranded polynucleotide segment hybridizable with a first portion of the target nucleic acid and attached to the label moiety; and
(h) a second polynucleotide marker probe comprising a second single-stranded polynucleotide segment hybridizable with a second portion of the marker nucleic acid being bound to a second test zone separate from the reaction zone and the first test zone.

38. A method for determining a single-stranded HIV nucleic acid and for confirming the efficiency of amplification thereof in a sample containing a marker nucleic acid and suspected of containing HIV nucleic acid, which method comprises:
(a) contacting the sample in a reaction zone with HIV oligonucleotide primers having the following sequences 5′ TGTGGAGGGGAATTTTTCTACTGTAA 3′ and 5' TATATAATTCACTTCTCCAATTGTCCCTCAT 3'
(b) contacting the sample in the reaction zone with an oligonucleotide primer for each single-stranded marker nucleic acid whose copy number in the sample is known, which primer is capable of annealing selectively to a complementary portion of the single-stranded marker nucleic acid;
(c) contacting the sample in the reaction zone with mononucleotides of each of the four nucleic acid bases;
(d) amplifying the copy number of the HIV and marker nucleic acids in the sample;
(e) contacting in the reaction zone, under conditions permissive of hybridization, the amplified copy number of the nucleic acid with a first HIV polynucleotide probe comprising a label moiety attached to a first single-stranded polynucleotide segment having an HIV-specific sequence selected from the group consisting of 5' CCTCCTCCAGGTCTGAAGATCTCGGACTCATTGTT 3' and 5' GTCCACTGATGGGAGGGGCATACATTGCTTTTCCT 3'
(f) permitting hybridization of the first HIV probe with any of the amplified copy number of nucleic acid present so as to form an HIV hybrid;
(g) contacting in the reaction zone, under conditions permissive of hybridization, the amplified copy number of marker nucleic acid with a first polynucleotide marker probe comprising a label moiety attached to a first single-stranded polynucleotide segment hybridizable with a first portion of the marker nucleic acid;
(h) permitting hybridization of the first marker probe with any of the amplified copy number of marker nucleic acid present so as to form a marker hybrid;
(i) contacting in a first test zone separate from the reaction zone an aliquot of the sample as treated in steps (a)-(f), under conditions permissive of hybridization, with a second polynucleotide HIV probe comprising a second single-stranded polynucleotide segment bound to the first test zone and having the other of the HIV probe sequences;
(j) contacting in a second test zone separate from the reaction zone and the first test zone a second polynucleotide marker probe comprising a single-stranded nucleic acid sequence hybridizable with a second portion of the marker nucleic acid and bound to the second test zone;
(k) permitting hybridization of the hybrids with the test zone-affixed second probes;
(l) separating, if necessary, nucleic acids bound to the test zones from any unbound nucleic acid; and
(m) determining the copy number of the amplified marker nucleic acid and the presence or absence of any HIV nucleic acid.

39. A composition for the determination of a plurality of single-stranded target nucleic acids in a sample to be tested, which composition comprises:
(a) an oligonucleotide primer for each single-stranded target nucleic acid of interest, each of which primers is capable of annealing selectively to a complementary portion of the single strands of target nucleic acid;
(b) mononucleotides of each of the four nucleic acid bases;
(c) nucleic acid polymerases capable of producing in a reaction zone extension products from the primers and mononucleotides, which extension products are complementary to the single strand of target nucleic acid to which a particular primer is annealed;
(d) a first polynucleotide probe for each of the nucleic acid targets comprising a label moiety and a first single-stranded polynucleotide segment attached to the label moiety, each of the first single-stranded polynucleotide segment being hybridizable with a first portion of its respective target nucleic acid;
(e) a second polynucleotide probe for each of the nucleic acid targets comprising a second single-stranded polynucleotide segment hybridizable with a second portion of its target nucleic acid and being bound to a test zone separate from the test zones for each of the other target nucleic acids.

40. A composition for determining a plurality of single-stranded target nucleic acids in a sample to be tested which comprises:
(a) an oligonucleotide primer for each single-stranded target nucleic acid of interest, each of which primers is capable of annealing selectively to a complementary portion of the single strands of target nucleic acid;
(b) mononucleotides of each of the four nucleic acid bases;
(c) nucleic acid polymerases capable of producing in a reaction zone extension products from the primers and mononucleotides, which extension products are complementary to the single strand of target nucleic acid to which a particular primer is annealed;
(d) a first polynucleotide probe for each of the nucleic acid targets comprising a label system component attached to a first single-stranded polynucleotide segment hybridizable with a first portion of its target nucleic acid;
(e) a second polynucleotide probe for each of the nucleic acid targets comprising a second single-stranded polynucleotide segment hybridizable with a second portion of its target nucleic acid and a specific binding entity attached to the second polynucleotide segment; and
(f) a plurality of test zones each separate from the reaction zone and from each of the other test zones and each having attached thereto a binding partner that will bind only with the specific binding entity attached to the second probe for the target nucleic acid of interest.

41. A composition for the determination of single-stranded target nucleic acid in a sample to be tested, which composition comprises:
(a) an oligonucleotide primer for each single-stranded target nucleic acid of interest, which primer is capable of annealing selectively to a complementary portion of the single strand of target nucleic acid;
(b) mononucleotides of each of the four nucleic acid bases;
(c) nucleic acid polymerases capable of producing an extension product from the primer and mononucleotides which is complementary to the single strand of target nucleic acid to which the primer is annealed to provide amplified nucleic acid sequences;
(d) a first polynucleotide probe comprising a first particle to which is attached a plurality of first single-stranded polynucleotide segments complementary to a first portion of the target nucleic acid; and
(e) a second polynucleotide probe comprising a second particle having attached thereto a plurality of second single-stranded polynucleotide segments complementary to a second portion of the target nucleic acid.

42. A method for the determination of single-stranded target nucleic acid in a sample to be tested, which method comprises the steps of:
(a) amplifying the copy number of the target nucleic acid;
(b) contacting, under conditions permissive of hybridization, the target nucleic acid with
(i) a first polynucleotide probe comprising a first particle to which is attached a plurality of first single-stranded polynucleotide segments complementary to a first portion of the target nucleic acid, and
(ii) a second polynucleotide probe comprising a second particle having attached thereto a plurality of second single-stranded polynucleotide segments complementary to a second portion of the target genetic material;
(c) permitting hybridization of the first polynucleotide segments to the first portion of target nucleic acid and the second segments to the second portion of target nucleic acid the hybridizations resulting in agglutination; and
(d) detecting the agglutination.
